(19) Europäisches Patentamt
European Patent Office
Office européen des brevets

(11) Publication number: **0 673 924 A1**

# (12) EUROPEAN PATENT APPLICATION published in accordance with Art. 158(3) EPC

(21) Application number: **94902092.9**

(51) Int. Cl.[6]: **C07C 279/18**, A61K 31/245

(22) Date of filing: **09.12.93**

(86) International application number:
**PCT/JP93/01783**

(87) International publication number:
**WO 94/13631 (23.06.94 94/14)**

(30) Priority: **10.12.92 JP 360711/92**
**12.11.93 JP 318909/93**

(43) Date of publication of application:
**27.09.95 Bulletin 95/39**

(84) Designated Contracting States:
**AT BE CH DE DK ES FR GB GR IE IT LI LU MC NL PT SE**

(71) Applicant: **TEIKOKU CHEMICAL INDUSTRY CO., LTD.**
**1-18, 1 chome, Kitahorie**
**Nishi-ku**
**Osaka-shi**
**Osaka 550 (JP)**

(72) Inventor: **MURAMATSU, Mutsumi 6-2, Iwasaki, Kitayamacho**
**Tokushima-shi**
**Tokushima 770 (JP)**
Inventor: **TAMURA, Toshiaki Itami Plant Teikoku**
**Chemical Industries Co., Ltd.**
**41, Senzo 5-chome**
**Itami-shi**
**Hyogo 664 (JP)**
Inventor: **YANAGI, Toshiharu Itami Plant Teikoku**
**Chemical Industries Co., Ltd.**
**41, Senzo 5-chome**
**Itami-shi**
**Hyogo 664 (JP)**

(74) Representative: **Harding, Charles Thomas et al**
**D. Young & Co.**
**21 New Fetter Lane**
**London EC4A 1DA (GB)**

## (54) PROPIONIC ACID DERIVATIVE.

(57) A propionic acid derivative represented by general formula (I) or a pharmaceutically acceptable acid-addition salt thereof, wherein A represents OH, $C_1$-$C_8$ lower alkoxy, -$NR_1R_2$, or $C_1$-$C_8$ lower alkoxy which may be substituted by halogen, optionally susbstituted aryl, -COB or succinimido; $R_1$ and $R_2$ represent each H, $C_1$-$C_8$ lower alkyl or optionally substituted aralkyl, or alternatively $R_1$ and $R_2$ are combined together with the adjacent nitrogen atoms to represent a heterocycle; and B represents OH, $C_1$-$C_8$ lower alkyl, optionally susbstituted aryl, optionally substituted aryloxy, $C_1$-$C_8$ lower alkoxy, optionally substituted aralkyloxy, or $NR_1R_2$ wherein $R_1$ and $R_2$ are each as defined above. This compound is useful as a protease inhibitor.

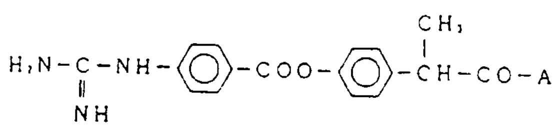 (I) (I)

EP 0 673 924 A1

TECHNICAL FEILD

This invention concerns nobel and useful compounds as enzyme inhibitiors, especially as active inhibitors for protease, protease inhibitors comprising of the above compounds, and a pharmaceutical composition for protease inhibitors comprising of the compounds and pharmaceutically acceptable carriers. And the invention concerns also the method of teatment for disease caused by abnormal conditions of the enzymes to administer the effective dose of the compounds to patients or animal patients.

TECHNICAL BACKGROUND

Trypsin, chymotrypsin, plasmin and thrombin are included in the enzyme group classified as serine protease. It has been known that several diseases are occured by abnormal activations of these enzymes for some reasons. For example, thrombin is a chemotactic factor at leukyocytic migration period which is called the second period of inflammation, and is also related to a promotive factor of froliferation or mitosis promotive factor.

It has been also known that the histamine isolation from mast cell is suppressed by the inhibition of chymotripsin typed protease related to the histamine isolation.

It seems that the disorder of pancreas occures as a result of the activation of other digestive enzymes caused by the activation of trypsin, and the illness become worse when those enzymes started to flow out into blood.

Protease in the blood are related to a blood coagulation system, fibrinolysis system being the opposite system to the blood coagulation system and other systems. If the blood coagulation occures at somewhere, the enzyme activity of the blood coagulation system and the fibrinogenolysis system arise, and as a result, the circulatory disorder occures.

Thus the abnormal activation of protease is related to the cause of various disease.

Mesylic acid gabegisate (FOY), which is used for the treatment of pancreatitis, is a well-known medicine as a protease inhibitor which inhibits trypsin and kalikrein. It is a trypsin-like enzyme in the blood, and it seems that it has inhibition activity to thrombin which plays an important role in the blood coagulation system, and also to plasmin which is related to the fibrinolysis system.

Chemical synthesized protease inhibitor has a tendency to inhibit the similar enzymes generally strongly or weakly.

To use protease inhibitor for the treatment of dieseases, it is expected that the protease inhibitor has properties to function to only the abnormal enzymes as specifically as far as possible, and to calm down the abnormal enzymes. Compounds with such properties has been progressed now with trial and error. For example, Japanese Patent publication JP,B,57-14670, JP,B,61-1063, JP,A,55-115865, JP,A,62-155253, JP,A,62-103058 are already known.

Each summary of the above references is described as follows.

(1) Japanese Patent publication JP,B, 57-14670

This is the disclosure for the ester between p-guanidinobenzoic acid and p-dialkylcarbamoylmethoxycarbonylphenol. This compound shows inhibition activity to plasmin and trypsin. And it is effec tive for the treatment of hemorrhagic disease and acute pancreatitis.

(2) Japanese Patent publication JP,B,61-1063

This is the disclosure for the ester between p-guanidinobenzoic acid and 6-amidino-2-naphthol. This compound shows inhibition activity to trypsin, plasmin, kalikrein and thrombin. The inhibition activity to trypsin is effective for the treatment of pancreatitis. The inhibition activity to plasmin and kalikrein is effective for the treatment of hemorrhagic disease. The inhibition activity to thrombin is effective for the treatment of thrombus, and also effective for the anti-complement action of allergosis such as nephritis related to complement.

(3) Japanese Patent publication JP,A,55-115865

This is the disclosure for the ester between p-guanidinobenzoic acid and dialkylsulfamoylphenol. This compound shows inhibition ac tivity to trypsin and plasmin, and is effective for the treat ment of acute pancreatitis and hemorrhagic disease.

(4) Japanese Patent publication JP,A,62-155253

This is the disclosure for the ester between p-guanidinobenzoic acid and p-substitution alkylthiophenol. This compound shows in hibiton activity to trypsin, plasmin and thrombin. The inhibition activity to trypsin is effective for the treatment of pancreatitis. The in hibition activity to plasmin is effective for the treatment of hemorrhagic disease. And the inhibition activity to thrombin is effective for the treat ment of thrombus.

(5)Japanese Patent publication JP,A,62-103058

This is the disclosure for the ester between p-guanidinobenzoic acid and p-dialkylaminoalkylcarbamoyl substitution phenol. This compound shows inhibition activity to trypsin and thrombin, and is efective for the suppression of the blood coagualation and for the treatment of pancreatitis and intravascular coagulation syndrome.

If a nobel compound having more enzyme active inhibition activity than the above mentioned compounds as a protease inhibitors, could be provided, some disease caused by those enzymes can be treated effectively.

The purpose of this invention is to provide a nobel compound having superior inhibition activity in enzyme, especially in protease, in more detail, in the enzymes classified as serine protease.

Another purpose of this invention is to provide the phermaceutical composition which is effective for the treatment of some diseases caused by the abnormal state of the above mentioned enzymes, such as pancreatitis, hemorrhagic disease, thrombolysis, nephritis and disseminated intravascular coagulation, and also effective for the prevention of the coagulation of the perfusion blood at exchanging of dialysis and plasma.

And still more, other purpose of this invention is to provide the methods for the treatment of active inhibition of the above mentioned enzymes to administer the effective dose of the above mentioned compounds to patients and animal patients, or to provide the use of the above mentioned compounds for the preparetion of producing the above mentioned pharmaceutical composition.

DISCLOSURE OF THE INVENTION

This invention concerns propionic acid derivatives shown in formula(I) or the pharmaceutically acceptable salts as an useful proteolytic enzyme active inhibitor.

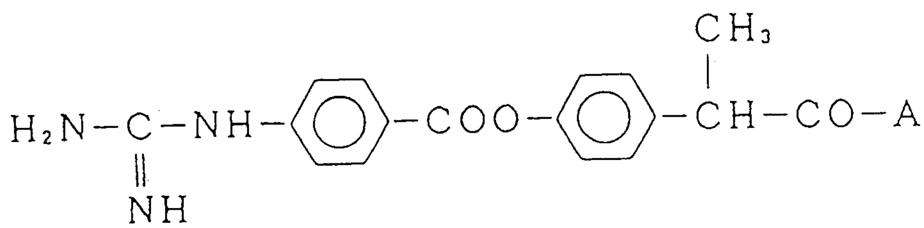

( I )

(In the formula, A is OH, the lower alkoxy of carbon number from one to eight, -N(R1,R2) or the lower alkoxy group of carbon number from one to eight which is substituted with halogen, aryl with or without substituents, group -COB or succinimide group. R1 and R2 is H, the lower alkyl of carbon number from one to eight, arylalkyl with or without subustituents, or a hetero cyclic ring together with the adjacent nitrogen atom. B is OH, the lower alkyl of carbon number from one to eight, aryl with or without substituents, aryloxy with or without substituents, the lower alkoxy of carbon number from one to eight, aralkyloxy with or without substituents or -N(R1,R2). ( here, R1 and R2 are the same as mentioned above. ))

In this invention, the lower alkoxy indicates alkoxy group of carbon number from one to eight, more, carbon number form one to four would be desirable. And the lower alkyl indicates alkyl group of carbon number from one to eight, more, carbon number from one to four would be desirable. And for example, there are phenyl group or naphtyl group for the aryl, and benzyl group or phenyl ethyl group for the arylalkyl.

And in this invention, the substituents of aryl or arylalkyl is, for examples, halogens such as fluorine, chloride, bromide and iodine, and the alkyl group of carbone number from one to eight such as methyl and ethyl. In the hetero cyclic ring together with the adjacent nitrogen atom, the ring size of them are membered five to seven rings. And the rings may have different atoms such as nitrogen, oxygen and sulfur. For

example, there are piperidine and piperazine allowing to have N-substituent ( for example, lower alkyl, benzyl, phenethyl and phenyl ) morpholine and pyrrolidine.

As the compound in this invention shown as the above mentioned formula(I) includes nitrogen atoms, it can produce various acis salt with various acids. These varous salts are also included in the compound in this invention shown as the above mentioned formula(I). These various salts with various acid are inorganic acid salt with HCl or HBr etc., organic sulfonate with methane sulfonic acid or p- toluenesulfonic acid etc., organic carboxylate or carbonate etc. with lactic acid, citric acid, succinic acid, tartaric acid, maleic acid or fumaric acid etc.

These various acid salt can be exchanged each others. It is possible to be exchanged each other directly, moreover, the way via carbonate is more desirable.

As the compound in this invention shown as the above mentioned formula(I) have an asymmetric carbon atom in the structure, it has a pair of optical enantiomer. As the comfigurations of the asymmeric carbon atom, namely, S-form, R-form, both of them and their racemates are included in the compound in this invention shown as the above mentioned formula(I).

The compound in this invention shown as the above mentioned formula(I) is produced by the estherification reaction between the compound shown as formula(II)

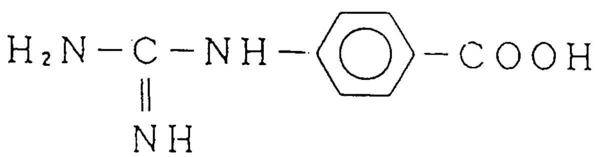

( II )

or the reactive derivatives of the compound and the propionic acid derivatives shown as formula(III).

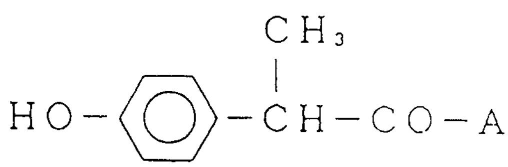

( III )

( A is the same as the definition of formula(I).)

The estherification reaction is achieved by mixing and stirring the compound shown as formula(II) or the reactive derivatives of the compound with the compound shown as formula(III) in a proper solvent. Condensation agents for the ester synthesis reaction such as dicyclohexylcarbodiimid, hydrogen chloride, sulfuric acid, may be used to make react the compound shown as formula(II) with the free -COOH to the compound shown as formula(III).

The reactive derivatives when using the reactive derivative of the compound shown as formula(II) are acid halide such as acid chloride and acid bromide, mixed acid anhydride with another acid derivative such as ethyl chlorcarbonate, acethyl chloride, and the reaction product with 1,1'-sulfinildiimidazol, 1,1'-carbonyldiimidazol.

The proper solvent for the reaction can be anything which does not react with the starting materials. The organic solvents such as ethyl ether, isopropyl ether, tetrahydrofuran, dioxane, dichlorethane, dichlormethane, acetonitrile, dimethylformamide, dimethylacetamide, pyridine, triethylamine, dimethylaminopyridine may be used. The reaction may be employed under either cooled or heated condition, which depend on the condition of the starting material.

The determination of using S-form or R-form of the formula(I) in this invention depends on which form, S-form or R-form, of the compound shown as formula(III) are used for the esterification reaction as a starting material with the compound shown as formula(II). Here, a racemate of the compound of formula(III) could be separated by the ordinary optical resolution. One of the favorable optical resolution is the way to make a diastereomer with an optically active base, and then separate the diastereomer. The favorable optically active bases are such as optically active 1- phenylehtylamine, optically active 1-(4-methylphenyl)-ethylamine, optically active 1-phenyl-2-methylaminopropane, optically active 1- phenyl-2-aminopropane, optically active bornyl amine, optically active menthyl amine, optically active 2-amino-1-oxyhydrolindane, L-arginine, L-lysine,chinchonine and chinchonidine.

For example, the optical resolution is to make a salt by reacting a racemate of 2-(4-hydroxyphenyl)-propionic acid with R-(+)-1- phenylethylamine($[\alpha]_D^{20}$ = +40.6) in solvent of aromatic hydorcaobon( for example, benzene, tholuene, xylene, etc. ), to take out the salt, and to separate it into dissolubles and insolubles in ketone solvent ( for example, acetone, methylethylketone, etc.). The insolubles in the keton solvent are collected, acidified with HCl and extracted with organic solvent( for example, methyl acetate, ethyl acetate, ethyl ether and isopropylether etc.). And, so (S)-(+)-2-(4-hydroxyphenyl)propionic acid is obtained.

On the other hand, the dissolubles in the keton solvent are concentrated under reduced pressure, and added aromatic solvent, and deposit chrystals are filtrated and collected. The deposit chrystals are acidified with HCl and extracted with organic solvent. And a salt was produced by reacting (S)-(-)-1-phenylethylamine($[\alpha]_D^{20}$ = -40.7) in solvent of aromatic hydrocarbon, and insoluble crystals in the keton solvent ( for example, acetone, methylethylketone, etc. ) are obtained by the same way as mentioned above. The obtained insoluble crystals are acidified with HCl and extracted with the organic solvent. And so, (R)-(-)-2-(4-hydroxyphenyl)propionic acid is obtained.

In the above resolution, the order of the reagent choice of which optical activative form of 1-phenylehtylamine to act on first does not matter. And so R-form or S-form of 2-(4-hydroxyphenyl)propionic acid can be obtained in the very good optical purity.

According to the ordinary procedure of esterification, various ester compounds included in formula (III) may be synethesized by using either the obtained (S)-(+)-2-(4-hydroxyphenyl) propionic acid or (R)-(-)-2-(4-hydroxyphenyl)propionic acid.

And this invention concerns the racemate, the R-form and the S-form of the above mentioned compound shown as formula (III) which is an useful intermediate to produce the compound shown as formula (I).

Also this invention concerns the protease inhibitor comprising of the propionic acid derivertives shown as formula (I) or the acid addition salt of it, and the pharmaceutical composition for protease inhibitor comprising of the propionic acid derivertives shown as formula (I) or the pharmaceutically acceptable acid adducted salt of it and a pharmaceutically acceptable carrier.

The compound of the above mentioned formula (I) obtained from this invention is administered as a pharmaceutical composition or, more concretely, as a pharmaceutical composition for protease inhibitor. The composition can be used in pharmaceutical forms such as tablets, capsules, powder, syrup or drysyrup, etc.

And the above mentioned pharmaceutical oral administration such as tablets, capsules, powder are obtained by adding the compound shown above as formula (I) as an active component, and excipients, bind ing agents, decay agents, glossing agents, lubricants, coloring agents, flavoring agents besides, and by sharping each agents' form by the usual procedure.

In this invention, the excipients are such as milk sugar, crystal cellulose, corn, starch, mannitol, etc., and the bonding agents are such as polyvinylpyrrolidone, hydroxypropylcellulose, carboxymethylcellulose, etc, and the decay agents are such as potato starch, sodium gluconate starch, calcium carboxy methylcellulose, etc, and the glossing agents are such as magnecium stearate, tarc, silica, etc, and the lubricants are such as sodium lauryl sulfate, syrup, starch, saponin, etc. To make the injection of the compound shown as formula(I) in this invention or the pharmaceutically acceptable addition salt of the compound is to dissolve the prescribed dose of the compound shown as formula(I) in distlled water for injection, and then to charge the obtained solution into amples.

The oral administration dose of the compound of formula(I) in this invention should be 10-300mg/day. And by making the pharmaceutical forms such as tablets including the proper amount of the dose, the compound can be prescribed once or several times as the oral administration. And when the compound is administered as injection, the compound shown as formula(I) should be administered 1- 50mg/day by phleboclysis.

The concrete examples of proteolytic enzyme of each inventions mentioned above are such as trypsin, kimotrypsin, plasmin, thrombin.

The enzyme inhibition of the compound shown as formula (I) presented by this invention was measured in the following way.

1. TRYPSIN INHIBITION

21.8mg of bnzoyl-DL-alginine-p-nitroanilide was dissolved in 2ml of dimethylsulfoxide, and 48ml of 0.1M boric acid buffer(pH8.0) including 5mM of calcium chloride added to it. And this mixure was used as substrate solution.

On the other hand, the enzyme solution of 3mg of trypsin dissolved in 10ml of 0.1M boric acid buffer including 5mM of calcium chloride was prepared.

The subject compound was diluted stepwise with the same buffer solution, and the solution of prescribed concentration was prepared. The mixture of 1ml of the substrate solution, 0.2ml of the enzyme solution and 1.8ml of the subject compound solution, was incubated for 10 minutes at 25°C. And 1ml of 30% acetic acid was added to the mixture to stop the enzyme reaction. The amount of hydrolyzed substrate was acquired by measuring the absorption at 410nm by using Erlangers' method. The concentration of the subject compound ( mol concentration ) to inhibit 50% amount of the substrate was acquired from the relation between the subject compound concentration and the hydrolyzed substrate amount. The value of IC50( the concentration of the subject compound to inhibit 50% amount of the substrate ) indicates the inhibition activity.

2. THROMBIN INHIBITION

Boc-Val-Pro-Arg-NH-Mec was dissolved in 0.1M boric acid buffer(pH8.0), and the concentration of 100 μM solution was prepared. And this was used as the substrate solution. On the other hand, a solution of 5ml of 500U thrombin ( Mochida Permaceutical CO. ) dissoluved in 0.1M boric acid buffer (pH8.0) was diluted 2000 times. And this was used as the enzyme solution.

The subject compound was diluted stepwise with the same buffer solution, and the solution of prescribed concentration was prepared. The mixture of 0.5ml of the substrate solution, 20 μl of the enzyme solution and 0.98ml of the subject compound solution was incubated for 10 minutes at 37°C. And 1ml of 30% acetic acid was added to the mixture to stop the enzyme reaction. The amount of hydrolyzed substrate was acquired by measuring the fluorescent strength of the fluorescence of 7-amino-4-methylcoumarin produced from the reaction, at the excitation wavelength of 380nm and the detection wavelength of 440nm. The concentration of the subject compound ( mol concentration ) to inhibit 50% amount of the substrate was acquired from the relation between the subject compound concentration and the hydrolyzed substrate amount. The value of IC50 ( the concentration of the subject compound to inhibit 50% amount of the substrate ) indicates the inhibition activity.

3. PLASMIN INHIBITION

Boc-Val-Leu-Lys-NH-Mec was dissolved in 0.1M boric acid buffer solution(pH8.0) including 0.15M sodium chloride, and the concentration of 20 μM solution was prepared, and was used as the substrate solution.

On the other hand, a solution of 1mg of Vridase ( Lederle Ltd. ) dissolved in 0.1M boric acid buffer solution including 0.15M sodium chloride was used as the streptokinase solution. Also, a solution of 5U plasminogen ( Sigma Ltd. ) dissolved in 0.1M boric acid buffer solution including 0.15M sodium chloride was diluted 50 times, and the solution was used as the enzyme solution. The subject compound was diluted stepwise with the same buffer solution, and the solution of prescribed concentration was prepared. 0.1ml of the enzyme solution was added to 0.1ml of the streptokinase solution, and it was incubated for 10 minutes at room temperature. After that, 0.75ml of the substrate solution and 0.55ml of the subject solution were added to the mixutre and incubated for 30 minutes at 37°C. And 1ml of 30% acetic acid was added to the mixture to stop the enzyme reaction. The amount of hydrolyzed substrate was acquired by measuring the fluorescent strength of the fluorescence of 7-amino-4-methylcoumarin produced from the reaction at the excitation wavelength of 380nm and the detection wavelength of 440nm. The concentration of the subject compound ( mol concentration ) to inhibit 50% amount of the substrate was acquired from the relation between the subject compound concentration and the hydrolyzed substrate amount. The value of IC50 ( the concentration of the subject compound to inhibit 50% amount of the substrate ) indicates the character of inhibition activity.

The compound of formula(I) presented by this invention includes the racemates and a pair of optically activated optical antipode, and it has a character to inhibit proteases. This compound is effective for treatment of diseases caused by the abnormal activation of the above enzymes such as acute pancreatitis, disseminated intravascular coagulation, hemodialysis. And it is also effective for the treatment of prevent ing the coagulation of the purfusion blood at exchanging of dialysis and plasma.

The compound of formula(I) in this invention can be used to make a pharmaceutical composition for a protease inhibition.

Although the concrete examples of this invention are described below, this invention is not ristricted to these examples.

THE BEST FORM TO CARRY OUT THE INVENTION

Although the concrete examples of this invention are described below, this invention is not ristricted to these concrete examples.

The preparation of the compound of formulla(III) is shown from example 1 to example 63. And the preparation of the compound of formula(I) is shown from example 64 to example 124. And also the preparation of pharmaceutical forms are shown from example 126 to example 129.

[Example 1]

2-(4-hydroxyphenyl)propionic acid methyl ester

6.44g of thionyl chloride was added dropwise to a solution of 3.00g of 2-(4-hydroxyphenyl)propionic acid in 20ml of methanol at ice bath temperature. The mixture was stirred for 1 hour at room temperature and concentrated under reduced pressure. Toluene and water were added to the residue and extracted with toluene. The organic layer of the extract was washed successively with water and saturated solution of sodium chloride, and then dried over anhydrous magnesium sulfate, and after concentrated under reduced pressure, 3.43g of oily products was obtained. It was purified by silica gel column chromatography with toluene-ethyl acetate eluent. The yield of the oily product, 2-(4-hydroxyphenyl)propionic acid methyl ester ( b.p.151-152 °C/3mmHg) was 3.23g.

The compounds of [Example 2] to [Example 4] of Table 1 were prepared in the same procedure described above, being only difference in using the cor responding alcohol.(make reference to table-1)

[Example 5]

2-(4-hydroxyphenyl)propionic acid benzyl ester

11.87g of triethylamine was added dropwise to a solution of 15.00g of 2-(4-hydroxyphenyl)propionic acid in 45ml of chloroform in an ice bath, and stirred for 30 minutes at room tempera ture. After the mixture was cooled in an ice bath, 18.53g of benzyl bromide was added dropwise, and the mixture was heated under reflux for 9 hours. The mixture was washed successively with water and saturated solution of sodium chloride, and then dried over an hydrous magnesium sulfate, after concentrated under reduced pressure, 21.86g of the oily product was obtwained. It was purified by silica gel column chromatogroaphy with toluene-ethyl acetate eluents. The yield of the oily product was 16.28g.

The compounds of [Example 6] and [Example 7] of Table 1 were prepared in the same procedure described above being difference in using benzyl bromide derivative for [Example 6](ref.table-1) and 1-bromo-2-succinimide ethane for [Example 7](make reference to table-1).

[Example 8]

2-(4-hydroxyphenyl)propionic acid N,N-dimethylcarbamoylmethyl ester

3.65g of triethylamine was added to a solution of 5.00g of 2-(4-hydroxyphenyl)propionic acid in 50ml of chlorloform at room temperature, and stirred for 30 minutes. After the mixture was cooled in an ice bath, 5.99g of α-bromo-N,N-dimethylacetoamide was added to this mixture, and then the mixture was heated under reflux for 9 hours. The mixture was washed successively with water and saturated solution of sodium chloride, and dried over anhydrous magnesium sulfate, after concentrated under reduced pressure, 8.62g of the oily product was obtained. It was purified by silica gel column chromatography with toluene-ethyl acetate eluent, and the product, 2-(4-hydroxyphenyl)propionic acid N,N-dimethylcarbamoylmethyl ester, were obtained as similar white colored crystals. The yield of the product was 5.89g.

The compounds of [Example 9] to [Example 17] of Table 1 and 2 were prepared in the same procedure described above using the corresponding α-bromoacetoamide derivatives(ref.table-1,2), the compounds of [Example 18] to [Example 22] of Table 2 and 3 using the corresponding α-bromoacetate derivatives-(ref.table-2,3), and the compounds of [Example 23] and [Example 24] of Table 3 using the corresponding α-bromoketone derivatives(ref.table-3), respectively.

[Example 25]

N,N-diethyl-2-(4-hydroxyphenyl)propioneamide

A drop of concentrated sulfulic acid was added to 55.13g of acetic anhydride in an ice bath. At 10-20°C, 2-(4- hydroxyphenyl)propionic acid was added to the solution, and stirred for 30 minutes at the same temperature. The mixture was added to 700ml of 1N hydrochloric acid, stirred for 1 hour at room temperature, and extracted with ethyl acetate. The organic layer of the extracted material was washed twice with saturated solution of sodium chloride, dried over anhydrous magnesium sulfate, and con centrated under reduced pressure. Isopropylether(30ml) was added to the residue, and the deposit crystals were collected by fil teration. The yield of 2-(4-acetoxyphenyl)propionic acid was 21.98g.
m.p.98-101°C
NMR ($CDCl_3$) δ
1.65(3H,d), 2.27(3H,s), 3.75(1H,q),
6.98(2H,d), 7.31(2H,d), 8.13(1H,bs)

2.91g of triethylamine was added dropwise to a solution of 5.00g of 2-(4-acetoxyphenyl)propionic acid in 25ml of dichloromethane in an ice bath,. After 2.86g of chloroethylcarbonate was added to the mixture at -30°C, it was warmed slowly up to -5°C. The mixture was cooled to -30°C again, 2.11g of diethylamine was added dropwise, and then warmed up slowly and stirred for 30 minutes in an ice bath. The mixture was washed successively with water, saturated sodium hydrogencarbonate solution, water, and 1N hydrochloric acid, and after concentrated under reduced pressure, 5.28g of the oily product was obtained. It was dissolved in 25ml of dried methanol, and cooled in an ice bath, then 4.26g of methanol solution containing 28% of sodium methylate was added. The mixture was stirred for 30 minutes and cooled in an ice bath. After water was added to the mixture, it was adjusted to pH2 with 6N hydrochloric acid, and extracted with ethyl acetate. The organic layer of the extracted product was washed with saturated sodium chloride solution, dried over anhydrous magnesium sulfate, and concentrated under reduced pressure. Isopropylether was added to the residue, and the deposit crystals of it were collected by filteration. The yield of the product, N,N-diethyl-2-(4-hydroxyphenyl)propioneamide, was 3.45g.

The compounds of [Example 26] to [Example 30] of Table 3 were obtained in the same procedure described above, being differnce in using the corresponding amine compounds.
(ref.table-3)

The properties of obtained products of Example 1 to 30 are described at table 1,2 and 3.

[Example 31]

In an ice bath, 15.03g of thionyl chloride was added drop wise to a solution of 7.00g of(S)-(+)-2-(4-hydroxyphenyl) propionic acid( $[\alpha]_D^{20}$ = + 70.3°(c=1.00, EtOH)) in 42ml of methanol. The mixture was stirred for 2 hours at the same temperature, and concentrated under reduced pressure. Toluene and water was added to the residue, and extracted with toluene. The organic layer of the extracted mterial was washed successively with water, sodium hydrogencarbonate solution, water, and saturated sodium chloride solution, dried over anhydrous magnesium sulfate, and concentrated under reduced pressure. Hexane was added to the residue, the deposit crystals were collected by filteration, and it was washed with hexane. The yield of(S)-(+)-2-(4- hydroxyphenyl)propionic acid methylester was 7.28g.
m.p.36-37°C
$[\alpha]_D^{20}$ = +91.8° (c=1.00, EtOH)
NMR ($CDCl_3$) δ
1.47(3H,d), 3.62(3H,s), 3.65(1H,q),
6.02(1H,s), 6.70(2H,d), 7.10(2H,d)

[Example 32]

In an ice bath, 10.74g of thionyl chloride was added drop wise to a solution of 5.00g of (S)-(+)-2-(4-hydroxyphenyl)propionic acid($[\alpha]_D^{20}$ = +70.3° (c=1.00, EtOH)) in 30ml of ethanol. The mixture was stirred for 10 minutes at the same temperature, and additional 2.5 hours at room temperature. After the mixture was concentrated under reduced pressure, toluene and water was added to the residue, and extracted with toluene. The organic layer was washed successively with water, sodium hydrogencarbonate solution, water, and saturated sodium chloride solution, dried over anhydrous magnesium sulfate, and concentrated under reduced pressure. Hexane was added to the residue, the deposit crystals were collected by filteration, and

they were washed with hexane. The yield of (S)-(+)-2-(4-hydroxyphenyl)propionic acid ethylester was 5.25g.
m.p.55-56°C
$[\alpha]_D^{20}$ = +65.2° (c=1.00, EtOH)
NMR ($CDCl_3$) δ
1.19(3H,t), 1.45(3H,d), 3.62(1H,q),
4.08(2H,q), 5.94(1H,s), 6.68(2H,d)
7.10(2H,d)

[Example 33]

In an ice bath, 10.74g of thionyl chloride was added drop wise to a solution of 5.00g of (R)-(-)-2-(4-hydroxyphenyl)propionic acid($[\alpha]_D^{20}$ = -70.2° (c=1.00, EtOH)) in 30ml of ethanol. The mixture was stirred for 40 minutes at the same temperature and additional 3 hours at room temperature. After the mixture was concentrated under reduced pressure, toluene and water was added to the residue, and extracted with toluene. The organic layer was washed successively with water, sodium hydrogencar bonate solution, water, and saturated sodium chloride solution, dried over anhydrous magnesium sulfate, and concentrated under reduced pressure. Hexane was added to the residue, the deposit crystals were collected by filteration, and they were washed with hexane. The yield of (R)-(-)-2-(4-hydroxyphenyl)propionic acid ethylester was 5.18g.
m.p.56-57°C
$[\alpha]_D^{20}$ = -66.0° (c=1.00, EtOH)
NMR ($CDCl_3$) δ
1.19(3H,t), 1.46(3H,d), 3.64(1H,q),
4.12(2H,q), 6.26(1H,s), 6.73(2H,d)
7.15(2H,d)

[Example 34]

In an ice bath, 15.03g of thionyl chloride was added drop wise to a solution of 7.00g of (S)-(+)-2-(4-hydroxyphenyl) propionic acid($[\alpha]_D^{20}$ = +70.3° (c=1.00, EtOH)) in 30ml of 2- propanol. The mixture was heated under reflux for 3 hours, and concentrated under reduced pressure. After toluene and water was added to the residue, the mixture was extracted with toluene. The organic layer was washed successively with water, sodium hydrogencarbonate solution, water, and saturated sodium chloride solution, dried over anhydrous magnesium sulfate, and con centrated under reduced pressure. Hexane was added to the residue, the deposit crystals were collected by filteration, and they were washed with hexane. The yield of (S)-(+)-2-(4- hydroxyphenyl)propionic acid isopropylester was 8.01g.
m.p.78-79°C
$[\alpha]_D^{20}$ = +46.7° (c=1.00, EtOH)
NMR($CDCl_3$) δ
1.13(3H,d), 1.20(3H,d), 1.44(3H,d),
3.61(1H,q), 5.00(1H,quin), 6.21(1H,s)
6.74(2H,d), 7.15(2H,d)

[Example 35]

At room temperature, 15.03g of thionyl chloride was added dropwise to a solution of 7.00g of (S)-(+)-2-(4-hydroxyphenyl) propionic acid($[\alpha]_D^{20}$ = +70.3° (c=1.00, EtOH)) in 34.5ml of 2,2,2-trifluoroethanol, and stirred overnight at the same temperature. After 17.16g of thionyl chloride was added to the mixture again, it was stirred for additional 5 hours, and concentrated under reduced pressure. Toluene and water was added to the residue, and extracted with toluene. The organic layer was washed successively with water, sodium hydrogencarbonate solution, water, and saturated sodium chloride solution, dried over anhydrous magnesium sulfate, concentrated under reduced pressure, and 9.32g of the residual oil was collected. It was purified by silica gel column chromatography with toluene-ethyl acetate eluent. The yield of (S)-(+)-2-(4-hydroxyphenyl) propionic acid 2,2,2-trifluoroethylester was 4.22g.
an oily substance.
$[\alpha]_D^{20}$ = +57.7° (c=1.00, EtOH)
NMR($CDCl_3$)δ
1.54(3H,d), 3.77(1H,q), 4.44(2H,q),

5.01(1H,s), 6.76(2H,d), 7.17(2H,d)

[Example 36]

At room temperature, 22.69g of benzyl bromide was added to a solution of 20.80g of (S)-(+)-2-(4-hydroxyphenyl)propionic acid sodium salt which was prepared from (S)-(+)-2-(4-hydroxyphenyl) propionic acid($[\alpha]_D^{20}$ = +70.3° (c=1.00, EtOH)) in 120ml of N,N- dimethylformamide. The mixture was stirred for 30 minutes and then overnight at 55-60°C. After the mixture was cooled in an ice bath, toluene and water was added, and it was extracted with toluene. The organic layer was washed successively with water, dilute hydrochloric acid, water, and saturated sodium chloride solution, dried over anhydrous magnesium sulfate, and concentrated under reduced pressure, and 35.90g of the residual oil was collected. It was purified by silica gel column chromatography with toluene-ethyl acetate eluent. The yield of (S)-(+)-2-(4-hydroxyphenyl)propionic acid benzylester was 20.90g.

m.p.79-80°C

$[\alpha]_D^{20}$ = +25.7° (c=1.00, EtOH)

NMR($CDCl_3$)δ

1.46(3H,d), 3.69(1H,q), 5.06(2H,s),
5.44(1H,s), 6.67(2H,d), 7.10(2H,d)
7.21(5H,s)

[Example 37]

At room temperature, 12.35g of benzyl bromide was added to a mixture of 10.00g of (R)-(-)-2-(4-hydroxyphenyl)propionic acid ($[\alpha]_D^{20}$ = -70.2° (c=1.00, EtOH)), 100ml of chloroform and 7.30g of triethylamine. After the mixture was heated under reflux for 15 hours, it was cooled in an ice bath. The mixture was washed successively three times with water, once with saturated sodium chloride solution, dried over anhydrous magnesium sulfate, con centrated underreduced pressure, and the residual oil was collected. It was purified by silica gel column chromatography with toluene-ethyl acetate eluents. The yield of(R)-(-)-2-(4-hydroxyphenyl)propionic acid benzylester was 7.43g.

m.p.80-81°C

$[\alpha]_D^{20}$ = -25.4° (c=1.00, EtOH)

NMR($CDCl_3$)δ

1.47(3H,d), 3.71(1H,q), 5.08(2H,s),
5.68(1H,s), 6.70(2H,d), 7.14(2H,d)
7.26(5H,s)

[Example 38]

At room temperature, 9.56g of 4-fluorobenzyl bromide was added to a mixture of 7.00g of (S)-(+)-2-(4-hydroxyphenyl)propionic acid ($[\alpha]_D^{20}$ = +70.3° (c=1.00, EtOH)), 70ml of chloroform and 5.11g of triethylamine. After the mixture was heated under reflux for 15 hours, it was cooled in an ice bath. The mixture was washed successively three times with water, once with saturated sodium chloride solution, dried over anhydrous magnesium sulfate, concentrated under reduced pressure, and 9.95g of the residual oil was collected. It was purified by silica gel column chromatography with toluene-ethyl acetate eluent. The yield of (S)-(+)-2-(4-hydroxyphenyl)propionic acid 4-fluorobenzyl ester was 6.33g.

m.p.74-74.5°C

$[\alpha]_D^{20}$ = +25.6° (c=1.00, EtOH)

NMR($CDCl_3$)δ

1.47(3H,d), 3.72(1H,q), 5.07(2H,s),
5.93(1H,s), 6.7-7.5(8H,m)

[Example 39]

At room temperature, 10.41g of 1-bromo-2-succinimide ethane was added to a mixture of 7.00g of (S)-(+)-2-(4-hydroxyphenyl) propionic acid ($[\alpha]_D^{20}$ = +70.3° (c=1.00, EtOH)), 70ml of chloroform and 5.11g of triethylamine. After the mixture was heated under reflux for 35 hours, it was cooled in an ice bath. The mixture was washed successively three times with water, once with saturated sodium chloride solution, dried over anhydrous magnesium sulfate, concentrated under reduced pressure, and 13.64g of the residual

oil was collected. It was purified by silica gel column chromatography with toluene-ethyl acetate eluent. The yield of (S)-(+)-2-(4-hydroxyphenyl) propionic acid 2-succinimide ethyl ester was 10.33g.
m.p.96-97°C
$[\alpha]_D^{20}$ = +57.2° (c=1.00, EtOH)
NMR ($CDCl_3$) δ
1.42(3H,d), 2.56(4H,s), 3.59(1H,q),
3.73(2H,bt), 4.23(2H,bt), 6.47(1H,bs),
6.73(2H,d), 7.10(2H,d)

[Example 40]

At room temperature, 5.99g of α-bromo-N,N-dimethylacetoamide was added to a mixture of 5.00g of (S)-(+)-2-(4- hydroxyphenyl)propionic acid ($[\alpha]_D^{20}$ = +70.3° (c=1.00, EtOH)), 50ml of chloroform and 3.65g of triethylamine. After the mixture was heated under reflux for 10 hours, it was cooled in an ice bath. The mixure was washed successively three times with water, once with saturated sodium chloride solution, dried over an hydrous magnesium sulfate, and concentrated under reduced pressure. After hexane was added to the residue, the deposit crystals were collected by filteration. They were washed with hexane, and 6.78g of the crude crystals were collected. They were purified by recrystallization from 2-propanol. The yield of (S)-(+)-2-(4-hydroxyphenyl) propionic acid N,N-dimethylcarbamoyl methyl ester was 5.19g.
m.p.121-122°C
$[\alpha]_D^{20}$ = +80.9° (c=1.00, EtOH)
NMR($CDCl_3$)δ
1.50(3H,d), 2.92(6H,s), 3.79(1H,q),
4.63(1H,d), 4.72(1H,d), 6.72(2H,d),
7.70(1H,s), 7.15(2H,d)

[Example 41]

At room temperature, 5.99g of α-bromo-N,N-dimethylacetoamide was added to a mixture of 5.00g of (R)-(-)-2-(4- hydroxyphenyl)propionic acid ($[\alpha]_D^{20}$ = -70.2° (c=1.00, EtOH)), 50ml of chloroform and 3.65g of triethylamine. After the mixture was heated under reflux for 10.5 hours, it was cooled in an ice bath. The mixture was washed successively three times with water, once with saturated sodium chloride solution, dried over anhydrous magnesium sulfate, and concentrated under reduced pres sure. After hexane was added to the residue, the deposit crystals were collected by filteration. They were washed with hexane, and 6.91g of the crude crystals were collected. They were purified by recrystallization from 2-propanol. The yield of (R)-(-)-2-(4-hydroxyphenyl) propionic acid N,N-dimethylcarbamoyl methyl ester was 5.65g.
m.p.122-123°C
$[\alpha]_D^{20}$ = -80.2° (c=1.00, EtOH)
NMR($CDCl_3$)δ
1.49(3H,d), 2.90(3H,s), 2.93(3H,s),
3.77(1H,q), 4.62(1H,d), 4.74(1H,d),
6.73(2H,d), 7.14(2H,d), 7.54(1H,bs)

[Example 42]

At room temperature, 7.68g of α-bromo-N-methylacetoamide was added to a mixture of 7.00g of (S)-(+)-2-(4-hydroxyphenyl) propionic acid ($[\alpha]_D^{20}$ = +70.3° (c=1.00, EtOH)), 70ml of chloroform and 5.11g of triethylamine. After the mixture was heated under reflux for 8 hours, it was cooled in an ice bath. The mixture washed successively three times with water, once with saturated sodium chloride solution, dried over anhydrous magnesium sulfate, concentrated under reduced pressure, and the residual oil was collected. It was purified by silica gel column chromatography with tholuene-ethyl acetate eluent. The yield of (S)-(+)-2-(4-hydroxyphenyl)propionic acid N-methylcarbamoylmethyl ester was 6.34g.
m.p.72-73°C
$[\alpha]_D^{20}$ = +75.3° (c=1.00, EtOH)
NMR($CDCl_3$)δ
1.50(3H,d), 2.62(3H,d), 3.73(1H,q),
4.42(1H,d), 4.69(1H,d), 5.65(1H,bs),
6.83(2H,d), 7.16(2H,d), 7.44(1H,s)

[Example 43]

At room temperature, 6.97g of α-bromoacetoamide was added to a mixture of 7.00g of (S)-(+)-2-(4-hydroxyphenyl)propionic acid ($[\alpha]_D^{20}$ = +70.3° (c=1.00, EtOH)), 70ml of chloroform and 5.11g of triethylamine. After the mixture was heated under reflux for 10 hours, it was cooled in an ice bath. The mixture was concentrated under reduced presssure, ethyl acetate and water was added to the residue, and extracted with ethyl acetate. The organic layer was washed successively with water, saturated sodium hydrogencarbonate solution and saturated sodium chloride solution, dried over anhydrous magnesium sulfate, and con centrated under reduced pressure again. Toluene was added to the residue, the deposit crystals were collected by filteration. They were washed with toluene and 6.57g of the crude crystals were collected. They were purified by recrystallization from 2-propanol. The yield of (S)-(+)-2-(4-hydroxyphenyl)-propionic acid carbamoylmethylester was 5.16g.
m.p.127-128°C
$[\alpha]_D^{20}$ = +83.4° (c=1.00, EtOH)
NMR($CDCl_3$)δ
1.39(3H,d), 3.73(1H,q), 4.28(1H,d),
4.43(1H,d), 6.66(2H,d), 7.09(2H,d),
7.10(2H,bs), 9.12(1H,s)

[Example 44]

At room temperature, 9.81g of α-bromo-N,N-diethylacetoamide was added to a mixture of 7.00g of (S)-(+)-2-(4-hydroxyphenyl)propionic acid ($[\alpha]_D^{20}$ = +70.3° (c=1.00, EtOH)), 70ml of chloroform and 5.11g of triethylamine. After the mixture was heated under reflux for 15 hours, it was cooled in an ice bath. The mixture was washed successively twice with water, once with saturated sodium chloride solution, dried over anhydrous magnesium sulfate, concentrated under reduced pressure, and the residual oil was collected. It was purified by silica gel column chromatography with toluene-ethyl acetate eluant. The yield of (S)-(+)-2-(4-hydroxyphenyl)propionic acid N,N-diethylcarbamoyl methylester was 6.34g.
m.p.70-71°C
$[\alpha]_D^{20}$ = +76.9° (c=1.00, EtOH)
NMR($CDCl_3$)δ
1.14(6H,bt), 1.49(3H,d), 3.28(4H,bq),
3.78(1H,q), 4.57(1H,d), 4.78(1H,d),
6.74(2H,d), 7.12(2H,d), 7.77(1H,bs)

[Example 45]

At room temperature, 12.24g of α-bromo-N-benzyl-N-methylacetoamide was added to a mixture of 7.00g of (S)-(+)-2-(4- hydroxyphenyl)propionic acid ($[\alpha]_D^{20}$ = +70.3° (c=1.00, EtOH)), 70ml of chloroform and 5.11 g of triethylamine. After the mixture was heated under reflux for 8.5 hours, it was cooled in an ice bath. The mixture was washed successively three times with water, once with saturated sodium chloride solution, dried over anhydrous magnesium sulfate, concentrated under reduced pressure, and the residual oil was collected. It was purified by silica gel column chromatography with toluene-ethyl acetate eluent. The yield of (S)-(+)-2-(4-hydroxyphenyl)propionic acid N-benzyl-N-methylcarbomoylmethyl ester was 11.72g.
an oily substance
$[\alpha]_D^{20}$ = +67.8° (c=1.27, EtOH)
NMR($CDCl_3$)δ
1.49(3H,d), 2.85(3H,br), 3.78(1H,m),
4.3-4.7(2H,m), 4.57(1H,d), 4.85(1H,d),
6.70(2H,d), 7.09(1H,d), 7.10(2H,d),
7.21(5H,bs)

[Example 46]

At room temperature, 9.71g of α-bromopyrrolidinoacetoamide was added to a mixture of 7.00g of (S)-(+)-2-(4-hydroxyphenyl) propionic acid ($[\alpha]_D^{20}$ = +70.3° (c=1.00, EtOH)), 70ml of chloroform and 5.11g of triethylamine. After the mixutre was heated under reflux for 14 hours, it was cooled in an ice bath. The mixture was washed successively twice with water, once with saturated sodium chloride solution, dried over

anhydrous magnesium sulfate, concentrated under reduced pressure, and the residual oil was collected. It was purified by silica gel column chromatography with toluene-ethyl acetate eluent. The yield of (S)-(+)-2-(4-hydroxyphenyl)propionic acid pyrrolidinocarbamoyl methylester was 9.12g.
m.p.93-94°C
$[\alpha]_D^{20}$ = +75.0° (c=1.00, EtOH)
NMR($CDCl_3$)δ
1.47(3H,d), 1.6-2.2(4H,m), 3.0-3.7(4H,m),
3.76(1H,q), 4.54(2H,s), 6.72(2H,d),
7.10(2H,d), 7.76(1H,bs)

[Example 47]

At room temperature, 10.42g of α-bromopiperidinoacetoamide was added to a mixture of 7.00g of (S)-(+)-2-(4-hydroxyphenyl) propionic acid ($[\alpha]_D^{20}$ = +70.3° (c=1.00, EtOH)), 70ml of chloroform and 5.11g of triethylamine. After the mixture was heated under reflux for 17 hours, it was cooled in an ice bath. The mixture was washed successively twice with water, once with saturated sodium chloride solution, dried over anhydrous mag nesium sulfate, concentrated under reduced pressure, and the residual oil was collected. It was purified by silica gel column chromatography with toluene-ethyl acetate eluent. The yield of (S)-(+)-2-(4-hydroxyphenyl)propionic acid piperidinocarbomoyl methylester was 10.12g.
m.p.81-82°C
$[\alpha]_D^{20}$ = +69.2° (c=1.00, EtOH)
NMR($CDCl_3$)δ
1.48(3H,d), 1.54(6H,bs), 3.23(2H,bs),
3.48(2H,bs), 3.77(1H,q), 4.58(1H,d),
4.77(1H,d), 6.74(2H,d), 7.14(2H,d), 7.53(1H,bs)

[Example 48]

At room temperature, 10.52g of α-bromomorpholinoacetoamide was added to a mixture of 7.00g of (S)-(+)-2-(4-hydroxyphenyl)propionic acid ($[\alpha]_D^{20}$ = +70.3° (c=1.00, EtOH)), 70ml of chloroform and 5.11g of triethylamine. After the mixture was heated under reflux for 6 hours, it was cooled in an ice bath. The mixture was washed successively twice with water, once with saturated sodium chloride solution, dried over anhydrous magnesium sulfate, and concentrated under reduced pressure. Afier 2-propanol was added to the residue, the deposit crystals were collected by filteration, and they were washed with 2-propanol. The yield of (S)-(+)-2-(4-hydroxyphenyl)propionic acid morpholino carbomoylmethylester was 10.51g.
m.p.115-116°C
$[\alpha]_D^{20}$ = +68.3° (c=1.00, EtOH)
NMR($CDCl_3$)δ
1.49(3H,d), 3.37(4H,bs), 3.58(4H,bs),
3.77(1H,q), 4.66(2H,s), 6.72(2H,d),
7.10(1H,bs), 7.12(2H,d)

[Example 49]

At room temperature, 7.73g of methyl bromoacetate was added to a mixture of 7.00g of (S)-(+)-2-(4-hydroxyphenyl)propionic acid ($[\alpha]_D^{20}$ = +70.3° (c=1.00, EtOH)), 70ml of chloroform and 5.11g of triethylamine. After the mixture was heated under reflux for 10 hours, it was cooled in an ice bath. The mixture was washed successively three times with water, once with saturated sodium chloride solution, dried over anhydrous magnesium sulfate, concentrated under reduced pressure, and the residual oil was collected. It was purified by silica gel column chromatography with tholuene-ethyl acetate eluent. The yield of (S)-(+)-2-(4-hydroxyphenyl)propionic acid methoxycarbonyl methylester was 7.15g.
an oily substance
$[\alpha]_D^{20}$ = +76.4° (c=1.00, EtOH)
NMR($CDCl_3$)δ
1.50(3H,d), 3.68(3H,s), 3.77(1H,q),
4.57(2H,s), 5.80(1H,s), 6.71(2H,d),
7.16(2H,d)

[Example 50]

At room temperature, 6.03g of ethyl bromoacetate was added to a mixture of 5.00g of (S)-(+)-2-(4-hydroxyphenyl)propionic acid ($[\alpha]_D^{20}$ = +70.3° (c=1.00, EtOH)), 50ml of chloroform and 3.65g of triethylamine. After the mixture was heated under reflux for 10 hours, it was cooled in an ice bath. The mixture was washed suc cessively twice with water, once with saturated sodium chloride solution, dried over anhydrous magnesium sulfate, concentrated under reduced pressure, and the residual oil was collected. It was purified by silica gel column chromatography with toluene- ethyl acetate eluent. The yield of (S)-(+)-2-(4-hydroxyphenyl)propionic acid ethoxycarbonylmethylester was 4.82g.
m.p.41-42°C
$[\alpha]_D^{20}$ = +71.3° (c=1.00, EtOH)
NMR($CDCl_3$)δ
1.21(3H,t), 1.49(3H,d), 3.74(1H,q),
4.14(2H,q), 4.54(2H,s), 5.84(1H,s),
6.69(2H,d), 7.13(2H,d)

[Example 51]

At room temperature, 6.03g of ethyl bromoacetate was added to a mixture of 5.00g of (R)-(-)-2-(4-hydroxyphenyl)propionic acid ($[\alpha]_D^{20}$ = -70.2° (c=1.00, EtOH)), 50ml of chloroform and 3.65g of triethylamine. After the mixture was heated under reflux for 10.5 hours, it was cooled in an ice bath. The mixture was washed successively three times with water, once with saturated sodium chloride solution, dried over anhydrous magnesium sulfate, con centrated under reduced pressure, and the residual oil was col lected. It was purified by silica gel column chromatography with toluene-ethyl acetate eluent. The yield of (R)-(-)-2-(4- hydroxyphenyl)propionic acid ethoxycarbonylmethylester was 5.08g.
m.p.41-42°C
$[\alpha]_D^{20}$ = -71.6° (c=1.00, EtOH)
NMR($CDCl_3$)δ
1.22(3H,t), 1.50(3H,d), 3.77(1H,q),
4.17(2H,q), 4.56(2H,s), 6.22(1H,s),
6.71(2H,d), 7.15(2H,d)

[Example 52]

At room temperature, 9.86g of t-buthyl bromoacetate was added to a mixture of 7.00g of (S)-(+)-2-(4-hydroxyphenyl) propionic acid ($[\alpha]_D^{20}$ = +70.3° (c=1.00, EtOH)), 70ml of chloroform and 5.11g of triethylamine. After the mixture was heated under reflux for 10 hours, it was cooled in an ice bath. The mixture was washed successively twice with water, once with saturated sodium chloride solution, dried over anhydrous magnesium sulfate, concentrated under reduced pressure, and the residual oil was collected. It was purified by silica gel column chromatography with toluene-ethyl acetate eluent. The yield of (S)-(+)-2-(4-hydroxyphenyl)propionic acid t-buthoxycarbonylmethyl ester was 7.32g.
m.p.64-65°C
$[\alpha]_D^{20}$ = +63.2° (c=1.00, EtOH)
NMR($CDCl_3$)δ
1.44(9H,s), 1.50(3H,d), 3.74(1H,q),
4.44(2H,s), 5.59(1H,s), 6.69(2H,d),
7.13(2H,d)

[Example 53]

At room temperature, 23.16g of benzyl bromoacetate was added to a mixture of 14.00g of (S)-(+)-2-(4-hydroxyphenyl)propionic acid ($[\alpha]_D^{20}$ = +70.3° (c=1.00, EtOH)), 140ml of chloroform and 10.22g of triethylamine. After the mixture was heated under reflux for 20 hours, it was cooled in an ice bath. The mixture was washed successively twice with water, once with saturated sodium chloride solution, dried over anhydrous magnesium sulfate, concentrated under reduced pressure, and the residual oil was collected. It was purified by silica gel column chromatography with toluene-ethyl acetate eluent. The yield of (S)-(+)-2-(4-hydroxyphenyl)propionic acid benzyloxycarbonylmethylester was 20.18g.
an oily substance.

$[\alpha]_D^{20}$ = +61.5° (c=1.00, EtOH)
NMR($CDCl_3$)δ
1.50(3H,d), 3.77(1H,q), 4.64(2H,s), 5.17(2H,s), 5.37(1H,s), 6.74(2H,d), 7.20(2H,d), 7.35(5H,s)

[Example 54]

At room temperature, 12.29g of 2,4-dimethylphenyl bromoacetate was added to a mixture of 7.00g of (S)-(+)-2-(4- hydroxyphenyl)propionic acid ($[\alpha]_D^{20}$ = +70.3° (c=1.00, EtOH)), 70ml of chloroform and 5.11g of triethylamine. After the mixture was heated under reflux for 10 hours, it was cooled in an ice bath. The mixture was washed successively twice with water, once with saturated sodium chloride solution, dried over anhydrous magnesium sulfate, concentrated under reduced pressure, and the residual oil was collected. It was purified by silica gel column chromatography with toluene-ethyl acetate eluent. The yield of (S)-(+)-2-(4-hydroxyphenyl) propionic acid 2,4-dimethylphenoxy carbonylmethylester was 8.51g.
m.p.96-98°C
$[\alpha]_D^{20}$ = +72.3° (c=1.00, EtOH)
NMR($CDCl_3$)δ
1.51(3H,d), 2.09(3H,s), 2.27(3H,s), 3.80(1H,q), 4.81(2H,s), 6.68(2H,d), 6.6-7.3(6H,m)

[Example 55]

At room temperature, 7.69g of bromoacetone was added to a mixture of 7.00g of (S)-(+)-2-(4-hydroxyphenyl)propionic acid ($[\alpha]_D^{20}$ = +70.3° (c=1.00, EtOH)), 70ml of chloroform and 5.11g of triethylamine. After the mixture was heated under reflux for 10 hours, it was cooled in an ice bath. The mixture was washed successively three times with water, once with saturated sodium chloride solution, dried over anhydrous magnesium sulfate, concentrated under reduced pressure, and the residual oil was col lected. It was purified by silica gel column chromatography with toluene-ethyl acetate eluent. The yield of (S)-(+)-2-(4-hydroxyphenyl)propionic acid methylcarbonylmethyl ester was 8.60g.
an oily substance.
$[\alpha]_D^{20}$ = +74.1° (c=1.00, EtOH)
NMR($CDCl_3$)δ
1.51(3H,d), 1.88(3H,s), 3.79(1H,q), 4.61(2H,s), 6.33(1H,s), 6.77(2H,d), 7.20(2H,d)

[Example 56]

At room temperature, 10.06g of 2-bromoacetophenone was added to a mixture of 7.00g of (S)-(+)-2-(4-hydroxyphenyl)propionic acid ($[\alpha]_D^{20}$ = +70.3° (c=1.00, EtOH)), 70ml of chloroform and 5.11g of triethylamine. After the mixture was heated under reflux for 12 hours, it was cooled in an ice bath. The mixture was washed successively twice with water, once with saturated sodium chloride solution, dried over anhydrous magnesium sulfate, concentrated under reduced pressure, and the residual oil was collected. It was purified by silica gel column chromatography with tholuene-ethyl acetate eluent. The yield of (S)-(+)-2-(4- hydroxyphenyl)propionic acid phenylcarbonylmethylester was 8.89g.
m.p.99-100°C
$[\alpha]_D^{20}$ = +84.6° (c=1.00, EtOH)
NMR($CDCl_3$)δ
1.54(3H,d), 3.85(1H,q), 5.24(1H,d), 5.31(1H,d), 6.17(1H,bs), 6.73(2H,d), 7.18(2H,d), 7.3-8.0(5H,m)

[Example 57]

In an ice bath, 6.44g of thionyl chloride was added dropwise to a solution of 3.00g of (R)-(-)-2-(4-hydroxyphenyl)propionic acid ($[\alpha]_D^{20}$ = -70.2° (c=1.00, EtOH)) in 20ml of 2-propanol. After the mixture was

heated under reflux for 3 hours, it was concentrated under reduced pressure. Toluene and water were added to the residue, and the mixture was extracted with toluene. The organic layer was washed successively with water, sodium hydrogen carbonate solution, water and saturated sodium chloride solution, dried over anhydrous magnesium, and concentrated under reduced pressure. Hexane was added to the residue, and the deposit crystals were collected by filteration, washed with hexane. The yield of (R)-(-)-2-(4-hydroxyphenyl)propionic acid isopropylester was 3.36g.
m.p.80-81 ° C
$[\alpha]_D^{20}$ = -47.9 ° (c = 1.00, EtOH)
NMR($CDCl_3$)δ
1.12(3H,d), 1.21(3H,d), 1.44(3H,d),
3.58(1H,q), 4.95(1H,quin), 6.17(1H,bs),
6.69(2H,d), 7.10(2H,d)

[Example 58]

At room temperature, 2.48g of α-bromo-N,N-diethylacetoamide was added to a solution of 2.00g of (R)-(-)-2-(4- hydroxyphenyl)propionic acid sodium salt, prepared from (R)-(-)- 2-(4-hydroxyphenyl)propionic acid ($[\alpha]_D^{20}$ = -70.2 ° (c = 1.00, EtOH)), in 15ml of N,N-dimethylformamide. The mixture was stirred for 30 minutes at the same temperature and then overnight at 50-60 ° C. The mixture was cooled, ethyl acetate and water were added, and extracted with ethyl acetate. The organic layer was washed with water, dried over anhydrous magnesium sulfate, concentrated under reduced pressure, and the residual oil was collected. It was purified by silica gel column chromatography with tholuene-ethyl acetate eluent. The yield of (R)-(-)-2-(4-hydroxyphenyl) propionic acid N,N-dimethylcarbamoylmethylester was 2.42g.
m.p.71.5-72 ° C
$[\alpha]_D^{20}$ = -77.7 ° (c = 1.00, EtOH)
NMR($CDCl_3$)δ
1.11(6H,bt), 1.47(3H,d), 3.29(4H,bq),
3.76(1H,q), 4.58(1H,d), 4.80(1H,d),
6.75(2H,d), 7.15(2H,d), 7.64(1H,s)

[Example 59]

At room temperature, 2.45g of α-bromopyrrolidinoacetoamide was added to a solution of 2.00g of (R)-(-)-2-(4- hydroxyphenyl) propionic acid sodium salt, prepared from (R)-(-)- 2-(4-hydroxyphenyl)propionic acid ($[\alpha]_D^{20}$ = -70.2 ° (c = 1.00, EtOH)), in 15ml of N,N-dimethylformamide. The mixture was stirred for 30 minutes at the same temperature and then overnight at 50-60 ° C. The mixture was cooled, ethyl acetate and water were added, and extracted with ethyl acetate. The organic layer was washed with water, dried over anhydrous magnesium sulfate, concentrated under reduced pressure, and the residual oil was collected. It was purified by silica gel column chromatography with toluene-ethyl acetate eluent. The yield of (R)-(-)-2-(4-hydroxyphenyl) propionic acid pyrrolidinocarbamoylmethylester was 1.89g.
m.p.95-97 ° C
$[\alpha]_D^{20}$ = -75.7 ° (c = 1.00, EtOH)
NMR($CDCl_3$)δ
1.47(3H,d),1.6-2.2(4H,m), 3.0-3.7(4H,m),
3.74(1H,q), 4.55(2H,s), 6.67(2H,d),
7.08(2H,d), 7.47(1H,bs)

[Example 60]

At room temperature, 2.49g of t-buthylbromoester acetate was added to a solution of 2.00g of (R)-(-)-2-(4- hydroxyphenyl) propionic acid sodium salt, prepared from (R)-(-)- 2-(4-hydroxyphenyl)propionic acid (-$[\alpha]_D^{20}$ = -70.2 ° (c = 1.00, EtOH)), in 15ml of N,N-dimethylformamide. The mixture was stirred for 30 minutes at the same temperature and overnight at 50-60 ° C. The mixture was cooled, ethyl acetate and water were added, and extracted with ethyl acetate. The organic layer was washed with water, dried over anhydrous magnesium sulfate, concentrated under reduced pressure, and the residual oil was collected. It was purified by silica gel column chromatography with toluene-ethyl acetate eluent. The yield of (R)-(-)-2-(4-hydroxyphenyl) propionic acid t-buthoxycarbonylmethylester was 2.61 g.
m.p.64-65 ° C

$[\alpha]_D^{20}$ = -63.8° (c=1.00, EtOH)
NMR($CDCl_3$)δ
1.43(9H,s), 1.50(3H,d), 3.73(1H,q),
4.47(2H,s), 5.83(1H,bs), 6.69(2H,d),
7.12(2H,d)

[Example 61]

At room temperature, 2.54g of 2-bromoacetophenone was added to a solution of 2.00g of (R)-(-)-2-(4-hydroxyphenyl) propionic acid sodium salt, prepared from (R)-(-)-2-(4- hydroxyphenyl) propionic acid ($[\alpha]_D^{20}$ = -70.2° (c=1.00, EtOH)), in 15ml of N,N-dimethylformamide. The mixture was stirred for 30 minutes at the same temperature and then overnight at 50-60°C. The mixture was cooled, ethyl acetate and water were added, and extracted with ethyl acetate. The organic layer was washed with water, dried over anhydrous magnesium sulfate, concentrated under reduced pressure, and the residual oil was collected. It was purified by silica gel column chromatography with toluene-ethyl acetate eluent. The yield of (R)-(-)-2-(4-hydroxyphenyl) propionic acid phenylcarbonylmethyester was 2.21g.
m.p.100-101°C
$[\alpha]_D^{20}$ = -84.9° (c=1.00, EtOH)
NMR($CDCl_3$)δ
1.54(3H,d), 3.84(1H,q), 5.29(2H,s),
6.02(1H,bs), 6.76(2H,d), 7.19(2H,d),
7.3-8.1(5H,m)

[Example 62]

At room temperature, 250g of 2-(4-hydroxyphenyl)propionic acid was added separately to a solution of 190.89g of (R)-(+)-1- phenylethylamine($[\alpha]_D^{20}$ = +40.6°) in 1250ml of toluene. The mixture was stirred for 0.5 hours at room temperature and for addi tional 1 hour at 90-95°C of internal temperature. After the mixture was stirred for 1 hour in an ice bath, the deposit crystals were collected by filteration, and they were washed with toluene. The yield of 2-(4-hydroxyphenyl) propionic acid (R)-(+)-1- phenylethylamine salt was 426.9g.
m.p.124-134°C

[Example 63]

A solution of 200g of 2-(4-hydroxyphenyl)propionic acid (R)- (+)-1-phenylethylamine salt in 1800ml of acetone was heated under reflux for 1 hour. While inoculating, the mixture was cooled down slowly, and then stirred for 1 hour at 25°C of internal temperature. After the deposit crystals were collected by filteration, they were washed with aceton, and 71.44g of similar white colored crystals(HPLC 96.6%ee) were collected. They were dissolved in 285ml of acetone, heated under reflux for 1 hour, and stirred for 1 hour at 20°C of internal temperature. The deposit crystals were collecetd by filteration, and they were washed with acetone. The yield of (S)-(+)-2-(4-hydroxyphenyl)propionic acid (R)-(+)-1-phenylethylamine salt (m.p.151-153°C $[\alpha]_D^{20}$ = +14.6° (c=1.00, EtOH), HPLC 98.2%ee) was 65.52g.

In an ice bath, a mixture of 60g of (S)-(+)-2-(4- hydroxyphenyl)propionic acid (R)-(+)-1-phenylethylamine salt, 300ml of ethyl acetate and 150ml of water was adjusted to pH2 with 23.98g of concentrated hydrochloric acid. The organic layer was separated by extraction, and then the aqueous layer was extracted with ethyl acetate again. The organic layer was added to the obtained aqueous layer, washed with water, dried over anhydrous magnesium sulfate. It was concentrated under reduced pressure, and 33.68g of crystal was collected. It was purified by recrystallization from water, and crystals were collected as needles. The yield of (S)-(+)-2-(4-hydroxyphenyl) propionic acid was 31.82g.
$[\alpha]_D^{20}$ = +70.3° (c=1.00, EtOH), HPLC98.99%ee, m.p. 161-165°C
NMR(DMSO-$d_6$)δ
1.34(3H,d), 3.53(1H,q), 6.69(2H,d),
7.08(2H,d), 9.2-12.2(2H,br)

Those two mother liquors of acetone were concentrated under reduced pressure. Toluene was added to the residue, dispersed, and 111.9g of similar white colored crystals were collected by filteration. They were dissolved in 500ml of ethyl acetate and 200ml of water, cooled in an ice bath, and the solution was adjusted to pH2 with 44.39g of concentrated hydrochloric acid. The organic layer was separated by extraction, and

then the aqueous layer was extracted with ethyl acetate again. The organic layer was added to the obtained aqueous layer, washed with water, dried over anhydrous magnesium sulfate, and concentrated under reduced pressure. Hexane was added to the residue, dispersed, and 61.46g of crystal(HPLC 60.2%ee) was collected by filteration. At room temperature, 60g of the crystal was added separately to a solution of 45.81g of (S)-(-)-1-phenylethylamine($[\alpha]_D^{20}$ = -40.7°) in 300ml of toluene. The mixture was stirred for 0.5 hours and for additional 1 hour at 85-90°C of internal temperature. The mixture was cooled in an ice bath, stirred for 1 hour, the deposit crystals were collected by filteration. They were washed with toluene, and 103.29g of crystal was collected. After it was dissolved in 410ml of acetone, it was heated under reflux for 1 hour, and then cooled. The mixture was stirred for 1 hour at 30°C, the deposit crysals were collected by filteration, and 70.88g of crystal(HPLC94.2%ee) was collected. It was dissolved in 280ml of acetone, heated undre reflux for 1 hour, and then cooled. The mixture was stirred for 1 hour at 25°C, the deposit crystals were collected by filteration, and washed with acetone. The yield of (R)-(-)-2-(4-hydroxyphenyl)propionic acid (S)-(-)-1- phenylethylamine salt(m.p.152-154°C $[\alpha]_D^{20}$ = -14.7° (c=1.00, EtOH), HPLC98.2%ee) was 61.94g.

In an ice bath, a mixture of 61.9g of (R)-(-)-2-(4- hydroxyphenyl)propionic acid (S)-(-)-1-phenylethylamine salt, 200ml of ethyl acetate and 100ml of water was adjusted to pH2 with 24.78g of concentrated hydrochloric acid. The organic layer was separated, and the aqueous layer was extracted with ethyl acetate again. The organic layer was added to the obtained aqueous layer, washed with water, dried over anhydrous magnesium sulfate, concentrated under reduced pressure, and 33.05g of crystal was collected. It was purified by recrystallization from water. The yield of (R)-(-)-2-(4-hydroxyphenyl) propionic acid was 31.40g.

$[\alpha]_D^{20}$ = -71.2° (c=1.00, EtOH), over99%ee in HPLC m.p. 165-166°C

NMR(DMSO-$d_6$)δ

1.34(3H,d), 3.54(1H.q), 6.73(2H,d),
7.12(2H,d), 9.2-12.1(2H,br)

HPLC measurement conditions
column Opti-PakTA ( Water's co.Ltd. )
mobile phase hexane : IPA : trifluoro acetic acid = 80:20:0.3
flow rate 1 ml/min.
detection UV 230nm

[Example 64]

At room tempearture, 3.44g of N,N-dicyclohexylcarbo diimide(DCC) was added to a mixture of 2.75g of 2-(4- hydroxyphenyl) propionic acid methyl ester, 3.00g of 4-guanidino benzoic acid hydrochloride and 30ml of pyridine. The mixture was stirred for 14 hour at room temperature, N,N-dimethylformamide(DMF) was added to the mixture, and some insoluble substances were filtered. After the mixture was concentrated under reduce pressure, ether was added to the residue by decantation. The mixture was dissolved in methanol, some insoluble substances were filtered, and concentrated under reduced pressure. The residual oil was dissolved in 150ml of water, filtered with celite. In an ice bath, 2.09g of potassium hydrogen carbonate solution was added separately to the filterate. After the mixture was stirred overnight at the same temperature, the deposit crystals were collected by filteration, washed with water and acetone, and 4.29g of its carbonate was collected. In an ice bath, 1.07g of methane sulfornic acid was added to a solution of the carbonate in acetone. After some insoluble subsatnces were filtered, the mixture was concentrated under reduce pressure. Hexane was added to the residue, the deposit crystals were collected by filteration, and they were washed with hexane. The yield of 2-[4-(4-guanidinobenzoyloxy) phenyl]propionic acid methyl ester • methane sulfonate was 3.80g.

This compound had 1/20 mol of acetone by NMR.
m.p. 151-155°C
NMR (DMSO-d6) δ

1.44(3H,d), 2.10(3/10H,s), 2.55(3H,s),
3.63(3H,s), 3.87(1H,q), 7.24(2H,d),
7.45(2H,d), 7.49(2H,d), 7.91(4H,bs),
8.21(2H,d), 10.36(1H,s)

enzyme inhibiting activity IC50 of trypsin 3.0 × 10-7
of thrombin 2.44 × 10-8

[Example 65]

At room tempearture, 3.44g of DCC was added to a mixture of 3.19g of 2-(4-hydroxyphenyl) propionic acid isopropyl esther, 3.00g of 4-guanidino benzoic acid hydrochloride and 20ml of pyridine. The mixture was stirred for 20 hour at room temperature, and DMF was added. After some insoluble substances were filtered, the mixture was concentrated under reduce pressure. Ether was added to the residue by decantation, and then dissolved in methanol. After some insoluble substances were filtered, the mixture was concentrated under reduced pressure. The residual oil was dissolved in 100ml of water, filtered with cerate, and 2.05g of potassium hydrogen carbonate solution was added separately to the filterate in an ice bath. After the mixture was stirred over night at the same temperature, the deposit crystals were collected by filteration. They were washed with water and acetone, and 3.64g of its carbonate was collected. In an ice bath, 0.85g of methane sulfornic acid was added to a solution of the carbonate in acetone, some insoluble subsatnces were filtered, and concentrated under reduce pressure. Hexane was added to the residue, the deposit crystals were collected by filteration, and they were washed with hexane. The yield of 2-[4-(4-guanidinobenzoyloxy)phenyl] propionic acid isopropyl ester • methane sulfonate was 2.85g.
m.p. 152-155 °C
NMR (DMSO-d6) $\delta$
1.13(3H,d), 1.20(3H,d), 1.43(3H,d),
2.58(3H,s), 3.77(1H,q), 4.94(1H,qq),
7.20(2H,d), 7.41(2H,d), 7.46(2H,d),
7.85(4H,bs), 8.19(2H,d), 10.29(1H,s)
enzyme inhibiting activity IC50 of trypsin 2.9 $\times$ 10-7
of thrombin 1.22 $\times$ 10-8

[Example 66]

At room tempearture, 3.44g of DCC was added to a mixture of 3.92g of 2-(4-hydroxyphenyl) propionic acid benzyl ester, 3.00g of 4-guanidino benzoic acid hydrochloride and 30ml of pyridine. After the mixture was stirred for 14 hour at room temperature, DMF was added, some insoluble substances were filtered, and concentrated under reduce pressure. Ether was added to the residue by decantation, dissolved in methanol, some insoluble substances were filtered, and concentrated under reduced pressure. The residual oil was dissolved in 100ml of water, filtered with celite, and 2.09g of potassium hydrogen carbonate solution was added separately to the filterate in an ice bath. After the mixture was stirred overnight at the same temperature, the deposit crystals were collected by filteration, washed with water and acetone, and 4.53g of its carbonate was collected. In an ice bath, 0.95g of methane sulfornic acid was added to a solution of the carbonate in acetone, some insoluble subsatnces were filtered, and concentrated under reduce pressure. After ethanol was added to the residue, the deposit crystals were filtered, and they were washed with ethanol. The yield of 2-[4-(4-guanidinobenzoyloxy) phenyl]propionic acid benzylester • methane sulfonate was 1.14g. This compound had 1/5mol of acetone by NMR.
m.p. 143.5-145 °C
NMR (DMSO-d6) $\delta$
1.46(3H,d), 2.10(1.2H,s), 2.49(3H,s),
3.90(1H,q), 5.10(2H,s), 7.27(5H,s),
7.41(2H,d), 7.82(4H,bs), 8.14(2H,d),
10.23(1H,s)
enzyme inhibiting activity IC50 of trypsin 3.7 $\times$ 10-7
of thrombin 5.4 $\times$ 10-8

[Example 67]

At room tempearture, 3.44g of DCC was added to a mixture of 3.31g of 2-(4-hydroxyphenyl) propionic acid 2,2,2-trifluoroethyl ester, 2.61g of 4-guanidino benzoic acid hydrochloride and 20ml of pyridine. After the mixture was stirred overnight at room temperature, DMF was added, some insoluble substances were filtered, and the mixture was concentrated under reduce pressure. Ether was added to the residue by decantation, dissolved in methanol, some insoluble substances were filtered, and the mixture was concentrated under reduced pressure again. The residual oil was dissolved in 100ml of water, filtered with celite, and 1.82g of potassium hydrogen carbonate solution was added separately to the filterate in an ice bath. After the mixture was stirred for two days in a refrigerator, the deposit crystals were collected by

filteration. They were washed with water and acetone, and 2.20g of its carbonate was collected. In an ice bath, 0.47g of methane sulfornic acid was added to a solution of the carbonate in acetone. After some insoluble substances were filtered, it was concentrated under reduce pressure. Hexane was added to the residue, the deposit crystals were collected by filteration, and they were washed with hexane. The yield of 2-[4-(4- guanidinobenzoyloxy)phenyl] propionic acid 2,2,2-trifluoroethyl ester • methane sulfonate was 2.36g.

m.p. 98-100 °C
NMR (DMSO-d6) δ
1.49(3H,d), 2.56(3H,s), 3.97(1H,q),
4.67(2H,q), 7.20(2H,d), 7.40(2H,d),
7.43(2H,d), 7.82(4H,bs), 8.15(2H,d),
10.02(1H,s)
enzyme inhibiting activity IC50 of trypsin 8.0 × 10-7
of thrombin 5.6 × 10-7

[Example 68]

At room tempearture, 3.44g of DCC was added to a mixture of 3.42g of 2-(4-hydroxyphenyl) propionic acid carbamoylmethyl ester, 3.00g of 4-guanidino benzoic acid hydrochloride and 20ml of pyridine. The mixture was stirred overnight at room temperature, DMF was added, some insoluble substances were filtered, and concentrated under reduce pressure. Ether was added to the residue by decantation, dissolved in methanol. After some insoluble substances were filtered, the mixture was concentrated under reduced pressure. The residual oil was dissolved in 100ml of water, filtered with celite, and 2.09g of potassium hydrogen carbonate solution was added separately to the filterate in an ice bath. After the mixture was stirred overnight at the same temperature, the deposit crystals were collected by filteration. They were washed with water and acetone, and 4.36g of its carbonate was collected. In an ice bath, 0.99g of methane sulfornic acid was added to a solution of the carbonate in methanol, some insoluble subsatnces were filtered, and concentrated under reduce pressure. After methanol and acetone were added to the residue, the deposit crystals were collected by filteration, and they were washed with acetone. The yield of 2-[4-(4-guanidinobenzoyloxy)phenyl] propionic acid carbamoylmethyl ester • methane sulfonate was 4.60g.

m.p. 166-170 °C
NMR (DMSO-d6) δ
1.48(3H,d), 2.54(3H,s), 3.5-4.3(2H,bs),
3.96(1H,q), 4.45(2H,bs), 7.19(2H,s),
7.40(2H,d), 7.43(2H,d), 7.80(4H,bs),
8.16(2H,d), 10.23(1H,s)
enzyme inhibiting activity IC50 of trypsin 2.0 × 10-7

[Example 69]

At room tempearture, 3.44g of DCC was added to a mixture of 3.85g of 2-(4-hydroxyphenyl) propionic acid N,N-dimethyl carbamoylmethyl ester, 3.00g of 4-guanidino benzoic acid hydrochloride and 20ml of pyridine. The mixture was stirred overnight at room temperature, DMF was added, some insoluble substances were filtered, and concentrated under reduce pressure. Ether was added to the residue by decantation, dissolved in methanol, some insoluble substances were filtered, and concentrated under reduced pressure. The residual oil was dissolved in 100ml of water, filtered with celite, and 2.09g of potassium hydrogen carbonate solution was added separately to the filterate in an ice bath. After the mixture was stirred overnight at the same temperature, the deposit crystals were collected by filteration, washed with water and acetone, and 4.82g of its carbonate was collected. In an ice bath, 0.98g of methane sulfornic acid was added to a solution of the carbonate in methanol, some insoluble subsatnces were filtered, and concentrated under reduce pressure. After ethanol and acetone were added to the residue, the deposit crystals were collected by filteration, and they were washed with acetone. The yield of 2-[4-(4-guanidinobenzoyloxy)phenyl]propionic acid N,N-dimethylcarbamoylmethyl ester • methane sulfonate was 3.38g.

m.p. 165-167 °C
NMR (DMSO-d6) δ
1.50(3H,d), 2.56(3H,s), 2.85(3H,s),
2.90(3H,s), 3.93(1H,q), 4.77(2H,s),

7.21(2H,d), 7.44(2H,d), 7.47(2H,d),
7.85(4H,bs), 8.18(2H,d), 10.33(1H,s)
enzyme inhibiting activity IC50 of trypsin 1.4 × 10-7

[Example 70]

At room tempearture, 2.30g of DCC was added to a mixture of 2.99g of 2-(4-hydroxyphenyl) propionic acid morpholinocarbonyl methyl ester, 2.00g of 4-guanidino benzoic acid hydrochloride, 7ml of pyridine and 14ml of DMF. The mixture was stirred overnight at room temperature, some insoluble substances were filtered, and concentrated under reduce pressure. Isopropylether(IPE) was added to the residue by decantation, dissolved in 100ml of water, filtered with celite. In an ice bath, 2.09g of potassium hydrogen carbonate solution was added separately to the filterate in an ice bath. The deposit crystals were collected by filteration, washed with water and acetone, and 1.37g of its carbonate was collected. In an ice bath, 0.28g of methane sulfornic acid was added to a solution of this carbonate in methanol, some insoluble subsatnces were filtered, concentrated under reduce pressure. Acetone and IPE were added to the residue, the deposit crystals were collected by filterataion, and they were washed with acetone. The yield of 2-[4-(4-guanidinobenzoyloxy) phenyl] propionic acid morpholinocarbonylmethyl ester • methane sulfonate was 1.06g.
m.p. 142-144 °C
NMR (DMSO-d6) δ
1.50(3H,d), 2.54(3H,s), 3.1-3.8(8H,m),
3.83(1H,q), 4.78(2H,s), 7.20(2H,d),
7.44(2H,d), 7.46(2H,d), 7.83(4H,bs),
8.15(2H,d), 10.26(1H,s)
enzyme inhibiting activity IC50 of trypsin 4.0 × 10-7
of thrombin 1.38 × 10-8

[Example 71]

At ice bath tempearture, 5.00g of 4-guanidinobenzoylchloride hydrochloride was added to a mixture of 5.33g of 2-(4- hydroxyphenyl)propionic acid 4-fluorobenzyl ester in 30ml of pyridine. The mixture was warmed slowly to room temperature, stirred overnight, and concentrated under reduce pressure. Ether was added to the residue by decantation, dissolved in 100ml of water. In an ice bath, a solution of 5.83g of potassium hydrogen carbonate in 50ml of water was added to the mixture. After the mixture was stirred for three hours at the same temperature, the deposit crystals were collected by filteration, washed with water and acetone, and 7.21g of its carbonate was collected. In an ice bath, 0.41g of methane sulfornic acid was added to a solution of 2.00g of the carbonate in acetone. After some insoluble substances were filtered, it was concentrated under reduce pressure. Ether and acetone were added to the residue, the deposit crystals were filtered, and they were washed with ether. The yield of 2- [4-(4-guanidinobenzoyloxy) phenyl]propionic acid 4-fluorobenzyl ester • methane sulfonate was 2.08g. This compound had 1mol of water by NMR.
m.p. 111-114 °C
NMR (DMSO-d6) δ
1.47(3H,d), 2.52(3H,s), 3.42(2H,bs),
3.87(1H,q), 5.06(2H,s), 6.8-7.5(8H,m),
7.39(2H,d), 7.75(4H,bs), 8.13(2H,d),
10.17(1H,s)
enzyme inhibiting activity IC50 of trypsin 6.8 × 10-7
of thrombin 3.3 × 10-9

[Example 72]

At ice bath tempearture, 5.00g of 4-guanidinobenzoylchloride hydrochloride was added to a mixture of 4.63g of 2-(4- hydroxyphenyl)propionic acid methoxycarbonylmethyl ester in 30ml of pyridine. The mixture was warmed slowly to room temperature, stirred overnight, and concentrated under reduce pressure. Ether was added to the residue by decantation, dissolved in 100ml of water, and a solution of 5.83g of potassium hydrogen carbonate in 50ml of water was added in an ice bath. After the mixture was stirred for two hours at the same temperature, the deposit crystals were collected by filteration, washed with water and acetone, and 7.45g of its carbonate was collected. In an ice bath, 0.44g of methane sulfornic acid was added to a

solution of 2.00g of the carbonate in acetone. After the deposit crystals were collected by filteration, they were washed with acetone. The yield of 2-[4-(4-guanidinobenzoyloxy)phenyl]propionic acid methoxycarbonylmethyl ester • methane sulfonate was 1.88g. The compound had 1mol of water by NMR.
m.p. 86-88 °C
NMR (DMSO-d6) δ
1.47(3H,d), 2.49(3H,s), 3.42(2H,bs),
3.62(3H,s), 3.93(1H,s), 4.64(2H,s),
7.16(2H,d), 7.37(2H,d), 7.40(2H,d),
7.76(4H,bs), 8.13(2H,d), 10.19(1H,s)
enzyme inhibiting activity IC50 of trypsin 3.8 × 10-7
of thrombin 1.64 × 10-7

[Example 73]

At ice bath tempearture, 5.00g of 4-guanidinobenzoylchloride hydrochloride was added to a mixture of 4.90g of 2-(4- hydroxyphenyl)propionic acid ethoxycarbonylmethylester in 30ml of pyridine. The mixture was warmed slowly up to room temperature, stirred overnight, and concentrated under reduce pressure. Ether was added to the residue by decantation, dissolved in 100ml of water. In a ice bath, a solution of 5.83g of potassium hydrogen carbonate in 50ml of water was added to the mixture. After the mixture was stirred for two hours at the same temperature, the deposit crystals were collected by filteration, washed with water and acetone, and 6.97g of its carbonate was collected. In an ice bath, 0.42g of methane sulfornic acid was added to a solution of 2.00g of the carbonate in acetone. After the deposit crystals were collected by filteration, concentrated under reduce pressure, they were washed with acetone. The yield of 2-[4-(4-guanidinobenzoyloxy) phenyl] propionic acid ethoxycarbonylmethylester • methane sulfonate was 1.21g. This compound had 1 mol of water by NMR.
m.p.121-124 °C
NMR (DMSO-d6) δ
1.17(3H,t), 1.51(3H,d), 2.47(3H,s),
3.36(2H,bs), 3.92(1H,q), 4.09(2H,q),
4.61(2H,s), 7.16(2H,d), 7.38(2H,d),
7.43(2H,d), 7.79(2H,bs), 8.14(2H,d), 10.22(1H,s)
enzyme inhibiting activity IC50 of trypsin 4.0 × 10-7
of thrombin 9.3 × 10-8

[Example 74]

At ice bath tempearture, 5.00g of 4-guanidinobenzoylchloride hydrochloride was added to a mixture of 5.44g of 2-(4- hydroxyphenyl)propionic acid t-buthoxycarbonylmethylester in 30ml of pyridine. The mixture was warmed slowly up to room temperature, stirred overnight, and concentrated under reduce pressure. Ether was added to the residue by decantation, dissolved in 100ml of water, and a solution of 5.83g of potassium hydrogen carbonate in 30ml of water was added in an ice bath. After the mixture was stirred for three hours at the same temperature, the deposit crystals were collected by filteration. They were washed with water and acetone, and 5.77g of its carbonate was collected. In an ice bath, 0.40g of methane sulfornic acid was added to a solution of 2.00g of carbonate in acetone, the deposit crystals were collected by filteration, acetone was added to the residue, the deposit crystals were collected by filteration, and they were washed with acetone. The yield of 2-[4-(4-guanidinobenzoyloxy)phenyl]propionic acid t-buthoxycarbonylmethyl ester • methane sulfonate was 1.54g. This compound had 1mol of water by NMR.
m.p.145-146 °C (decompsition)
NMR (DMSO-d6) δ
1.40(9H,s), 1.49(3H,d), 2.35(3H,s),
3.38(2H,bs), 3.90(1H,q), 4.48(2H,s),
7.16(2H,d), 7.37(2H,d), 7.40(2H,d),
7.74(4H,bs), 8.10(2H,d), 10.17(1H,s)
enzyme inhibitinh activity IC50 of trypsin 3.7 × 10-7
of thrombin 7.3 × 10-8

[Example 75]

At ice bath tempearture, 5.10g of 4-guanidinobenzoylchloride hydrochloride was added to a mixture of 6.22g of 2-(4- hydroxyphenyl)propionic acid benzyloxy carbonyl methyl ester in 30ml of pyridine. The mixture was warmed slowly up to room tem perature, stirred overnight, and concentrated under reduce pressure. Ether was added to the residue by decantation, dissolved in 100ml of water, and a solution of 5.96g of potassium hydrogen carbonate in 50ml of water was added in an ice bath. After the mixture was stirred for two hours at the same temperature, the reaction mixture was let stand overnight in a refrigerator. The deposit crystals were collected by filteration, washed with water and acetone, and 7.16g of its carbonate was collected. In an ice bath, 0.38g of methane sulfornic acid was added to a solution of 2.00g of the carbonate in acetone, some insoluble substances were filtered, and concentrated under reduced pressure. Acetone and ether were added to the residue, the deposit crystals were collected by filteration, and washed successively with acetone and ether. The yield of 2-[4-(4-guanidinobenzoyloxy) phenyl] propionic acid benzyloxycarbonylmethylester • methane sulfonate was 1.87g.

m.p. 105-107 °C

NMR (DMSO-d6) δ

1.47(3H,d), 2.49(3H,s), 3.92(1H,q),
4.69(2H,s), 5.12(2H,s), 7.13(2H,d),
7.31(5H,s), 7.37(2H,d), 7.47(2H,d),
7.78(4H,bs), 8.13(2H,d), 10.19(1H,s)

enzyme inhibiting activity IC50 of trypsin 6.1 × 10-7
of thrombin 9.3 × 10-8

[Example 76]

A solution of 2.00g of 2-[4-(4-guanidinobenzoyloxy)phenyl] propionic acid benzyloxycarbonylmethylester carbonate, obtained from [Example 75], in 40ml of acetic acid was hydrogenated in the presence of 0.20g of 10% Pd-C(contatining 48.3% of water) at ordinary temperature and pressure. After the catalyst was filtered, the mixture was concentrated under reduced pressure. Acetone was added to the residue, the deposit crystals were collected by filteration, washed with acetone, and 1.37g of crystals were collected. They were dissolved in 50ml of water, adjusted to pH8 with saturated sodium hydrogencarbonate solution. The deposit crystals were collected by filteration, and washed successively with water and acetone. In an ice bath, 0.36g of methane sufornic acid was added to a solution of the crystals in 30ml of methanol. After some insoluble substances were filtered, the mixture was concentrated under reduced pressure. Acetone and ether were added, the deposit crystals were filtered, and washed successively with acetone and ether. The yield of 2-[2-[4-(4-guanidinobenzoyloxy) phenyl] propyonyloxy] acetic acid • methane sulfonate was 1.25g.

m.p. 153-156 °C

NMR (DMSO-d6) δ

1.47(3H,d), 2.50(3H,s), 3.94(1H,q),
4.53(2H,s), 7.17(2H,d), 7.40(2H, d),
7.41(2H,d), 7.78(5H,bs), 8.15(2H,d),
10.20(1H,s)

enzyme inhibiting activity IC50 of trypsin 4.8 × 10-7
of thrombin 1.46 × 10-6

[Example 77]

At ice bath tempearture, 4.00g 4-guanidinobenzoylchloride hydrochloride was added to a solution of 4.02g of 2-(4- hydroxyphenyl)propionic acid benzylester and 24ml of pyridine. After warming the mixture up to room temperture, it was stirred overnight, and concentrated under reduced pressure. The residue was dissolved in 100ml of water, 4.69g of potassium hydrogen carbonate was added separately in an ice bath, and stirred overnight at the same temperature. The deposit crystals were collected by filteration, washed successively with water and acetone, and 7.25g of its carbonate was collected. A solution of 6.20g of the carbonate in 124g of acetic acid was hydrogenated in the presence of 0.62g of 10% Pd-C(containing 48.3% of water) at ordinary temperature and pressure. The catalyst was filtered, concentrated under reduced pressure. Acetone was added to the residue, filtered the deposit crystals, washed with acetone, and 3.59g of crystals were collected. The residue was dissolved in 40ml of methanol and water (methanol : water = 1

: 1), and adjusted to pH8 with saturated sodium hydrogen carbonate solution in an ice bath. After the mixture was stirred for 1.5 hours at the same temperature, the deposit crystals were collected by filteration. They were washed successively with water and acetone, and 3.39g of crystals were collected. In an ice bath, 1.00g of methane sulfornic acid was added to a solution of 3.24g of these crystals in methanol, some insolubles were filtered, and concentrated under reduced pres sure. Acetone and IPE were added to the residue, and concentrated under reduced pressure again. Acetone was added to the residue, the deposit crystals were collected by filteration, and washed successively with acetone and ether. The yield of 2-[4- (4-guanidino- benzoyloxy) phenyl] propionic acid • methane sulfonate was 2.81g.

m.p. 152-155 °C

NMR (DMSO-d6) δ

1.46(3H,d), 2.53(3H,s), 3.87(1H,q),
7.14(2H,d), 7.37(2H,d), 7.40(2H,d),
7.77(5H,bs), 8.13(2H,d), 10.20(1H,s),

enzyme inhibiting activity IC50 of trypsin 3.8 × 10-7
of thrombin 6.6 × 10-7

[Example 78]

At ice bath temperature, 5.67g of 4-guanidinobenzoylchloride hydrochloride in a mixture of 6.00g of 2-(4-hydroxyphenyl) propionic acid pyrrolidinocarbonylmethylester and 30ml of pyridine. After warming the mixture up to room temperture, it was stirred overnight, and concentrated under reduced pressure. In an ice bath, 6.60g of potassium hydrogen carbonate solution was added separately to a solution of the residue in 160ml of water. The deposit crystals were filtered, washed successively with water and acetone, and 8.30g of its caronate was collected. In an ice bath, 0.40g of methane sulfornic acid was added to a solu tion of 2.00g of the carbonate in acetone. After methanol was added to be homogeneous, some insoluble substances were filtered, and concentrated under reduced pressure. Actone was added to the residue, and concentrated under reduced pressure again. Ether was added to the residue, the deposit crystals were collected by filteration, and washed with ether. The yield of 2-[4-(4-guanidinobenzoyloxy)phenyl]propionic acid pyrrolidinocarbonyl methylester • methane sulfonate was 1.79g. This compound showed high hygroscopicity.

NMR (DMSO-d6) δ

1.47(3H,d), 1.6-2.1(4H,m), 2.51(3H,s),
3.21(4H,bt), 3.90(1H,q), 4.66(2H,s),
7.16(2H,d), 7.40(2H,d), 7.42(2H,d),
7.76(4H,bs), 8.13(2H,d), 10.20(1H,s)

enzyme inhibiting activity IC50 of trypsin 3.9 × 10-7
of thrombin 7.3 × 10-8

[Example 79]

At ice bath temperature, 7.00g of 4-guanidinobenzoylchloridehydrochloride was added to a mixture of 8.89g of 2-(4-hydroxyphenyl)propionic acid N-benzyl-N-methyl carbamoylmethylester and 40ml of pyridine. After warming the mixture up to room temperture, it was stirred overnight, and concentrated under reduced pressure. Ether was added to the residue by decantation, and dissolved in 100ml of water. In an ice bath, a solution of 8.16g of potassium hydrogen carbonate in 50ml of water was added to the mixture. The deposit crystals were dispersed, stirred for three hours at the same tempearture, and let stand overnight in a refrigerator. The deposit crystals were collected by filteration, washed with water, and 10.20g of its carbonate was collected. In an ice bath, 0.38g of methane sulfonic acid was added to a solution of 2.05g of the carbonate in methanol. After some insoluble substances were filtered, the mixture was concentrated under reduced pressure. Ether was added to the residue, the deposit crystals were collected by filteration, and they were washed with ether. The yield of 2-[4-(4-guanidinobenzoyloxy) phenyl]propionic acid N-benzyl-N-methylcarbamoylmethylester • methane sulfonate was 1.66g.

m.p. 109-111 °C

NMR (DMSO-d6) δ

1.51(3H,d), 2.53(3H,s), 2.85(3H,s),
3.94(1H,q), 4.51(2H,s), 4.89(2H,s),
7.20(2H,d), 7.27(5H,s), 7.45(2H,d),
7.46(2H,d), 7.78(4H,bs), 8.16(2H,d)

10.23(1H,s)
enzyme inhibiting activity IC50 of trypsin 3.9 × 10-7
of thrombin 7.3 × 10-8

[Example 80]

At ice bath temperature, 5.13g of 4-guanidinobenzoylchloride hydrochloride was added to a mixture of 3.87g of 2-(4- hydroxyphenyl)propionic acid ethylester and 30ml of pyridine. After warming the mixture up to room temperature, it was stirred overnight, and concentrated under reduced pressure. Ether was added to the residue by decantation, and dissolved in 100ml of water. In an ice bath, a solution of 5.98g of potassium hydrogen carbonate in 50ml of water was added dropwise to the mixture, and stirred for two hours at the same temperature. The deposit crystals were collected by filteration, washed successively with water and acetone, and 6.68g of its carbonate was collected. In an ice bath, 0.73g of methane sulfonic acid was added to a solution of 3.00g of the carbonate in acetone, some insoluble substances were filtered, and concentrated under reduced pressure. Ether was added to the residue, the deposit crystals were collected by filteration, washed with ether. The yield of 2-[4-(4-guanidinobenzoyloxy)phenyl]propionic acid ethylester • methane sulfonate was 2.91 g.
m.p. 135-137 °C
NMR (DMSO-d6) δ
1.16(3H,t), 1.44(3H, d), 2.54(3H,s),
3.80(1H,q), 4.09(2H,q), 7.23(2H,d),
7.40(2H,d), 7.44(2H,d), 7.84(4H,bs),
8.16(2H,d), 10.29(1H,s)
enzyme inhibiting activity IC50 of trypsin 3.3 × 10-7

[Example 81]

At ice bath temperature, 5.00g of 4-guanidinobenzoylchloride hydrochloride was added to a mixture of 5.42g of 2-(4- hydroxyphenyl)propionic acid N,N-diethylcarbamoylmethylester and 30ml of pyridine. After warming the mixture up to room temperature, it was stirred overnight, and concentrated under reduced pressure. Ether was added to the residue by decantation, and dissolved in 100ml of water. In an ice bath, a solution of 5.83g of potassium hydrogen carbonate in 50ml of water was added drop wise to the mixture, and stirred for two hours at the same tempearture. The deposit crystals were collected by filteration, washed successively with water and acetone, and 7.37g of its carbonate was collected. In an ice bath, 0.40g of methane sulfonic acid was added to a solution of 2.00g of the carbonate in acetone. While the mixture was homogeneous, some insoluble substances were filtered, and the filterate was let stand. The deposit crystals were collected by filteration, and they were washed with acetone. The yield of 2-[4-(4-guanidinobenzoyloxy) phenyl]propionic acid N,N-diethylcarbamoylmethylester • methane sulfonate was 1.64g.
m.p. 137-139 °C
NMR (DMSO-d6) δ
1.08(6H,t), 1.48(3H,d), 2.51(3H,s),
3.26(4H,bq), 3.92(1H,q), 4.74(2H,s),
7.19(2H,d), 7.42(2H,d), 7.44(2H,d),
7.82(4H,bs), 8.16(2H,d), 10.26(1H,s)
enzyme inhibiting activity IC50 of trypsin 2.8 × 10-7

[Example 82]

At ice bath temperature, 5.00g of 4-guanidinobenzoylchloride hydrochloride was added to a mixture of 5.66g of 2-(4- hydroxyphenyl)propionic acid piperidinocarbonylmethylester and 30ml of pyridine. After warming the mixture up to room temperature, it was stirred overnight, and concentrated under reduced pressure. Ether was added to the residue by decantation, and dissolved in 100ml of water. In an ice bath, a solution of 5.83g of potassium hydrogen carbonate in 50ml of water was added dropwise to the mixture, and stirred for three hours at the same ten, perature. The deposit crystals were collected by filteration, washed successively with water and acetone, and 4.84g of carbonate was collected. In an ice bath, 0.39g of methane sulfonic acid was added to a solution of 2.00g of carbonate in acetone, and methanol was added. While the solution was homogeneous, some insoluble substances were filtered, and concentratede under

reduced pressure. Acetone was added to the residue, and concentrated under reduced pressure again. Ether was added to the residue, the deposit crystals were collected by filteration, and they were washed with ether. The yield of 2-[4-(4- guanidinobenzoyloxy)phenyl]propionic acid piperidinocarbonyl methylester • methane sulfonate was 1.84g. This compound had 1/2mol of diethylether by NMR and showed high hygroscopicity.

NMR (DMSO-d6) δ

1.10(3H,t), 1.50(3H,d), 1.55(6H,bs),
2.53(3H,s), 3.33(4H,bs), 3.40(1.5H,q),
3.93(1H,q), 4.76(2H,s), 7.20(2H,d),
7.44(2H,d), 7.46(2H,d), 7.79(4H,bs)
8.17(2H,d), 10.24(1H,s)

enzyme inhibiting activity IC50 of trypsin 4.2 × 10-7

[Example 83]

At room temperature, 2.30g of DCC was added to a mixture of 2.97g of 2-(4-hydroxyphenyl) propionic acid 2-succinimide ethyl ester, 25ml of pyridine and 2.00g of 4-guanidinobenzoic acid hydrochloride. After the mixture was stirred overnight, DMF was added, some insoluble substances were filtered, and concentrated under reduced pressure. After ether was added to the residue by decantation, the mixture was dissolved in 100ml of water, and filtered with celite. In an ice bath, 1.39g of potassium hydrogencarbonate was added separately to the filterate, and stirried overnight in a refrigerator. The deposit crystals were collected by filteration, washed successively with water and acetone, and 2.74g of its carbonate was collected. In an ice bath, 0.54g of methane sufonic acid was added to a solution of the carbonate in methanol, some insoluble substances were filtered, and concentrated under reduced pressre. Acetone was added to the residue, and concentrated under reduced pressure again. Ether was added to the residue, the deposit crystals were collected by filteration, and washed with ether. The yield of 2-[4-(4-guanidinobenzoyloxy)phenyl]propionic acid 2- succinimide ethyl esther • methane sulfonate was 2.34g. This compound had 1/2mol of diethylether by NMR and showed high hygroscopicity.

NMR (DMSO-d6) δ

1.10(3H,t), 1.42(3H,d), 2.49(3H,s), 2.56(4H,s), 3.38(2H,q), 3.61(2H,bt),
3.75(1H,q), 4.16(2H,bt), 7.16(2H,d),
7.34(2H,d), 7.42(2H,d), 7.78(4H,bs),
8.14(2H,d), 10.17(1H,s)

enzyme inhibiting activity IC50 of trypsin 4.0 × 10-7

[Example 84]

At ice bath temperature, 5.00g of 4-guanidinobenzoylchloride hydrochloride was added to a mixture of 4.61g of 2-(4- hydroxyphenyl)propionic acid N-methylcarbamoylmethylester, 30ml of pyridine. After warming the mixture up to room temperature, it was stirred overnight, and concentrated under reduced pressure. Ether was added to the residue by decantation, and the mixture was dissolved in 100ml of water. In an ice bath, a solu tion of 5.83g of potassium hydrogen carbonate in 50ml of water was added dropwise to the mixture, and stirred for 1 hour at the same temperature. The deposit crystals were collected by filteration, washed successively with water and acetone, and 4.99g of carbonate was collected. and then methanol was added. While the solution was homogeneous, some insoluble substances were filtered, concentrated under reduced pressure, acetone was added to the residue, and concentrated under reduced pressure again. Ether was added to the residue, the deposit crystals were collected by filteration, and washed with ether. The yield of 2-[4-(4- guanidinobenzoyloxy)phenyl]propionic acid N-methylcarbamoyl methyl ester • methane sulfonate was 2.12g. This compound had 1/3mol of diethylether by NMR and showed high hygroscopicity.

NMR (DMSO-d6) δ

1.10(2H,t), 1.49(3H,d), 2.55(3H,s),
3.37(1.3H,q), 3.95(1H,q), 4.44(2H,bs),
7.16(2H,d), 7.40(4H,d), 7.77(4H,bs),
8.13(2H,d), 10.19(1H,s)

enzyme inhibiting activity IC50 of trypsin 3.9 × 10-7

[Example 85]

At ice bath temperature, 2.35g of 4-guanidinobenzoylchloride hydrochloride was added to a mixture of 3.00g of 2-(4- hydroxyphenyl)propionic acid 2,4-dimethylphenoxycarbonylmethyl ester and 20ml of pyridine. After warming the mixture slowly up to room temperture, it was stirred overnight, and concentrated under reduced pressure. Ether was added to the residue by decantation, and the mixture was dissolved in 100ml of water. In an ice bath, a solution of 5.83g of potassium hydrogencarbonate in 30ml of water was added droppwise to the mixture. After the mix ture was stirred for 1.5 hours at the same temperature, the deposit crystals were collected by filteration, They were washed successively with water and acetone, and 3.09g of carbonate was collected. In an ice bath, 0.37g of methane sulfonic acid was added to a soluiton of 2.00g of the carbonate in acetone. While the mixture was homogeneous, some insoluble substances were filtered, and the filterate was let stand. The deposit crystals were collected by filteration, and washed with acetone. The yield of 2-[4-(4-guanidinobenzoyloxy) phenyl]propionic acid 2,4-dimethylphenoxycarbonylmethyl ester • methane sulfonate was 1.91g.

m.p. 118-119.5 ° C

NMR (DMSO-d6) δ

1.50(3H,d), 2.06(3H,s), 2.25(3H,s),
2.36(3H,s), 3.97(1H,q), 4.93(2H,s),
6.7-8.4(11H,m), 7.78(4H,bs), 10.21(1H,s),

enzyme inhibiting activity IC50 of trypsin 6.2 × 10-7

[Example 86]

At ice bath temperature, 5.00g of 4-guanidinobenzoylchloride hydrochloride was added to a mixture of 4.32g of 2-(4- hydroxyphenyl)propionic acid methylcarbonylmethyl ester and 30ml of pyridine. After warming the mixture slowly up to room tem perature, it was stirred overnight, and concentrated under reduced pressure. Ether was added to the residue by decantation, and the mixture was dissolved in 100ml of water. In an ice bath, a solution of 5.83g of potassium hydrogencarbonate in 50ml of water was added dropwise to the mixture. After the mixture was stirred for 1 hour at the same temperature, the deposit crystals were collected by filteration. They were washed successively with water and acetone, and 5.98g of carbonate was collected. In an ice bath, 0.45g of methane sulfonic acid was added to a solu tion of 2.00g of the carbonate in acetone. After some insoluble substances were filtered, the mixture was concentrated under reduced pressure. Ether was added to the residue, and con centrated under reduced pressure again. Ether was added to the residue, the deposit crystals were collected by filteration, and they were washed with ether. The yield of 2-[4-(4- guanidinobenzoyloxy)phenyl]propionic acid methylcarbonylmethyl ester • methane sulfonate was 2.01g.

m.p. 139-141 ° C

NMR (DMSO-d6) δ

1.49(3H,t), 2.04(3H,s), 2.56(3H,s), 3.91(1H,q), 4.70(2H,s), 7.16(2H,d),
7.39(2H ,d), 7.44(2H,d), 7.80(4H,bs),
8.13(2H,d), 10.23(1H,s)

enzyme inhibiting activity IC50 of trypsin 4.1 × 10-7

[Example 87]

At ice bath temperature, 3.49g of 4-guanidinobenzoylchloride hydrochloride was added to a mixture of 3.00g of 2-(4- hydroxyphenyl)-N,N-diethylpropione amide and 30ml of pyridine. After warming the mixture slowly up to room temperature, it was stirred overnight, and concentrated under reduced pressure. Ether was added to the residue by decantation, and the mixture was dissolved in 100ml of water. In an ice bath, a solution of 4.07g of potassium hydrogencarbonate in 40ml of water was added dropwise to the solution. After the mixture was stirred for 1 hour at the same temperature, the deposit crystals were collected by filteration. They were washed successively with water and acetone, and 4.21 g of carbonate was collected. In an ice bath, 0.45g of methane sulfonic acid was added to a solution of 2.00g of the carbonate in acetone, some insoluble substances were filtered, and concentrated under reduced pressure. Ether was added to the residue, and concentrated under reduced pressure again. Ether was added to the residue, the deposit crystals were collected by filteration, and washed with ether. The yield of 4-guanidinobenzoic acid 4-[1-(N,N-diethylaminocarbonyl)ethyl] phenylester • methane sulfonate was 1.86g.

m.p. 153-155 ° C

NMR (DMSO-d6) δ
1.01(6H,bt), 1.35(3H,d), 2.53(3H,s),
3.29(4H,bq), 4.00(1H,q), 4.50(1H,bs),
7.16(2H,d), 7.39(2H,d), 7.42(2H,d),
7.78(4H,bs), 8.14(2H,d), 10.21(1H,s)
enzyme inhibiting activity IC50 of trypsin 3.8 × 10-7

[Example 88]

At ice bath temperature, 3.31g of 4-guanidinobenzoylchloride hydrochloride was added to a mixture of 3.00g of 2-(4- hydroxyphenyl)piperidinopropione amide and 30ml of pyridine. After warming the mixture slowly up to room temperature, it was stirred overnight, and concentrated under reduced pressure. Ether was added to the residue by decantation, and the mixture was dissolved in 100ml of water. In an ice bath, a solution of 4.07g of potassium hydrogencarbonate in 40ml of water was added dropwise to the mixture. After the mixture was stirred for 1 hour at the same temperature, the deposit crystals were collected by filteration, washed successively with water and acetone, and 3.97g of carbonate was collected. In an ice bath, 0.44g of methane sulfonic acid was added to a solution of 2.00g of the carbonate in acetone. After some insoluble substances were filtered, the mixture was concentrated under reduced pressure. Ether was added to the residue, and concentrated under reduced pressure again. Ether was added to the residue, the deposit crystals were collected by filteration, and they were washed with ether. The yield of 4-guanidino-benzoic acid 4-[1- (piperidinocarbonyl)ethyl]phenylester • methane sulfonate was 1.98g.
m.p. 161-163 °C
NMR (DMSO-d6) δ
1.34(3H,d), 1.43(6H,bs), 2.56(3H,s),
3.0-3.8(4H,m), 4.07(1H,q), 4.70(1H,bs),
7.13(2H,d), 7.34(2H,d), 7.39(2H,d),
7.81(4H,bs), 8.14(2H,d), 10.25(1H,s)
enzyme inhibiting activity IC50 of trypsin 4.4 × 10-7

[Example 89]

At ice bath temperature, 3.00g of 4-guanidinobenzoylchloride hydrochloride was added to a mixture of 2.25g of 2-(4- hydroxyphenyl)-N,N-dimethylpropione amide and 30ml of pyridine. After warming the mixture slowly up to room temperature, it was stirred overnight, and concentrated under reduced pressure. Ether was added to the residue by decantation, and the mixture was dissolved in 100ml of water. In an ice bath, a solution of 5.25g of potassium hydrogencarbonate in 70ml of water was added dropwise to the solution. After the mixture was stirred for two hours at the same temperature, the mixture was let stand over night in a refrigerator. The deposit crystals were collected by filteration, washed successively with water and acetone, and carbonate was collected. In an ice bath, 0.48g of methane sulfonic acid was added to a solution of 2.00g of the carbonate in acetone, and methanol was added. While the solution was homogeneous, some insoluble substances were filtered, and con centrated under reduced pressure. Acetone was added to the residue, the deposit crystals were collected by filteration, and washed successively with acetone and ether. The yield of 4- guanidinobenzoic acid 4-[1-(N,N-dimethylaminocarbonyl)ethyl] phenylester • methane sulfonate was 2.07g. This compound had 1mol of water by NMR.
m.p. 223-225 °C
NMR (DMSO-d6) δ
1.34(3H,d), 2.53(3H,s), 2.87(3H,s),
2.94(3H,s), 3.50(4H,bs), 4.10(1H,q),
7.14(2H,d), 7.36(2H,d), 7.41(2H,d),
7.76(4H,bs), 8.14(2H,d), 10.21(1H,s)
enzyme inhibiting activity IC50 of trypsin 3.4 × 10-7
of thrombin 1.2 × 10-7

[Example 90]

At ice bath temperature, 3.00g of 4-guanidinobenzoylchloride hydrochloride was added to a mixture of 2.97g of 2-(4- hydroxyphenyl)-N-methyl-N-phenylpropione amide and 30ml of pyridine. After warming the mixture slowly up to room temperature, it was stirred overnight, and concentrated under reduced pressure.

Ether was added to the residue by decantation, and the mixture was dissolved in 100ml of water. In an ice bath, a solution of 3.50g of potassium hydrogencarbonate in 50ml of water was added dropwise to the mixture. After the mixture was stirred for 2 hours at the same temperature, the mixture was let stand over night in a refrigerator. The deposit crystals were collected by filteration, washed successively with water, and 3.65g of carbonate was collected. In an ice bath, 0.42g of methane sulfonic acid was added to a solution of 2.00g of the carbonate in methanol. After some insoluble substances were filtered, the filterate was concentrated under reduced pressure. Ether was added to the residue, the deposit crystals were collected by filteration, and they were washed with ether. The yield of 4-guanidinobenzoylchloic acid 4-[1-(N-methyl-N-phenylaminocarbonyl) ethyl]phenylester • methane sulfonate was 2.01g. This compound had 1/6mol of diethylether and 1/2mol of water by NMR and didn't show clear melting point.

NMR (DMSO-d6) δ

1.11(1H,t), 1.33(3H,d), 2.54(3H,s),
3.17(3H,s), 3.40(2/3H,q), 3.70(1H,q),
4.60(1H,bs), 6.9-8.5(13H,m), 7.80(4H,bs),
10.26(1H,s)

enzyme inhibiting activity IC50 of trypsin 3.5 × 10-7
of thrombin 7.0 × 10-8

[Example 91]

At ice bath temperature, 3.00g of 4-guanidinobenzoylchloride hydrochloride was added to a mixture of 3.14g of 2-(4- hydroxyphenyl)-N-benzyl-N-methylpropione amide and 30ml of pyridine. After warming the mixture slowly up to room temperature, it was stirred overnight, and concentrated under reduced pressure. Ether was added to the residue by decantation, and the mixture was dissolved in 100ml of water. In an ice bath, a solution of 3.50g of potassium hydrogencarbonate in 50ml of water was added dropwise to the mixture. After the mixture was stirred for 1 hour at the same temperature, the mixture was let stand over night in a refrigerator. The deposit crystals were collected by filteration, washed successively with water and acetone, and 4.29g of carbonate was collected. In an ice bath, 0.41g of methane sulfonic acid was added to a solution of 2.00g of the carbonate in methanol. After some insoluble substances were filtered, the filterate was concentrated under reduced pressure. Ether was added to the residue, the deposit crystals were collected by filteration, and they were washed with ether. The yield of 4-guanidinobenzoic acid 4-[1-(N-methyl-N-benzylaminocarbonyl)ethyl]phenylester • methane sulfonate was 2.06g. This compound had 1/2mol of diethylether by NMR and didn't show clear melting point.

NMR (DMSO-d6) δ

1.10(3H,t), 1.40(3H,d), 2.56(3H,s),
2.71(3H,s), 3.41(2H,q), 4.15(1H,q),
4.57(1H,bs), 6.8-8.5(13H,m), 7.83(4H,bs),
10.32(1H,s)

enzyme inhibitinh activity IC50 of trypsin 4.0 × 10-7
of thrombin 3.1 × 10-8

[Example 92]

At ice bath temperature, 3.50g of 4-guanidinobenzoylchloride hydrochloride was added to a mixture of 2.25g of 2-(4- hydroxyphenyl)propione amide and 20ml of pyridine. The mixture was stirred for 1 hour at the same temperature and additional 3 hours at the room temperature, and then concentrated under reduced pressure. Ether was added to the residue by decantation, and the mixture was dissolved in 70ml of water. The solution was stirred for 1 hour at ice bath temperature, the deposit crystals were collected by filteration, and washed successivly with cold water and acetone. They were dissolved in isopropylalchol-(IPA), the deposit crystals were collected by filteration, and they were washed with IPA. The yield of 4-guanidinobezoic acid 4-[1-(aminocarbonyl)ethyl]phenylester • hydrochloride was 0.75g.

m.p.205-208 °C

NMR (DMSO-d6) δ

1.35(3H,d), 3.0-3.8(1H,bs), 3.68(1H,q),
6.79(1H,bs), 7.0-9.0(13H,m)

enzyme inhibiting activity IC50 of trypsin 3.0 × 10-7
of thrombin 6.0 × 10-8

[Example 93]

At ice bath temperature, 3.00g of 4-guanidinobenzoylchloride hydrochloride was added to a mixture of 3.31g of 2-(4- hydroxyphenyl)propionic acid phenylcarbonylmethylesther and 30ml of pyridine. After warming the mixture slowly up to room temperature, it was stirred overnight, concentrated under reduced pressure, and 11.61 g of residual oil was collected. Ether was added to it by decantation, and the mixture was dissolved in 100ml of water. In an ice bath, a solution of 3.50g of potassium hydrogencarbonate in 30ml of water was added dropwise to the solution. The solution was stirred for 2 hours at the same temperature, the deposit crystals were collected by filteration, washed successively with water and acetone, and 5.00g of carbonate was collected. In an ice bath, 0.40g of methane sulfonic acid was added to a solution of 2.00g of the carbonate in acetone. After some insoluble substances were filtered, the filterate was concentrated under reduced pressure. Acetone and ether were added successively to the residue, the deposit crystals were collected by filteration, and washed with ether. The yield of 2-[4-(4-guanidinobenzoyloxy)phenyl]-propionic acid phenylcarbonylmethylester • methane sulfonate was 1.80g. This compound had 1/20mol of diethylether by NMR.

m.p. 100-103 °C

NMR (DMSO-d6) $\delta$

1.10(0.3H,t), 1.37(3H,d), 2.57(3H,s),
3.39(0.2H,q), 3.99(1H,q), 5.43(2H,s),
7.0-8.5(17H,m), 10.26(1H,s)

enzyme inhibiting activity IC50 of trypsin 3.7 × 10-7
of thrombin 2.5 × 10-8

[Example 94]

At ice bath temperature, 2.02g of 4-guanidinobenzoylchloride hydrochloride was added to a mixture of 3.00g of 2-(4- hydroxyphenyl)propionic acid N-benzylpiperazinylcarbonylmethyl ester and 30ml of pyridine. After warming the mixture slowly up to room temperature, it was stirred overnight, and concentrated under reduced pressure. Ether was added to the residue by decantation, and the mixture was dissolved in 100ml of water. In an ice bath, a solution of 3.53g of potassium hydrogencarbonate in 50ml of water was added dropwise to the mixture. After the mixture was stirred for 2 hours at the same temperature, the deposit crystals were dispersed, and collected by filteration. They were washed with water, and 2.45g of carbonate was collected. In an ice bath, 0.50g of methane sulfonic acid was added to a solution of 1.50g of the carbonate in aectone, methanol was added. While the solution was homogeneous, some insoluble substances were filtered, and concentrated under reduced pressure. Acetone was added to the residue, and concentrated under reduced pressure again. Ether was added to the residue, and concentrated under reduced pressure. After ether was added to the mixture again, the deposit crystals were collected by filteration, and they were washed with ether. The yield of 2-[4-(4guanidinobenzoyloxy)phenyl]propionic acid N-benzylpiperazinyl carbonylmethylester • 2 methane sufonate was 1.58g. This compound had 1/6mol of diethylether by NMR and didn't show clear melting point.

NMR (DMSO-d6) $\delta$

1.12(1H,t), 1.50(3H,d), 2.57(6H,s),
2.9-4.0(8H,m), 3.42(2/3H,q), 3.93(1H,q),
4.37(2H,s), 4.87(2H,s), 5.30(2H,bs),
7.0-8.3(14H,m), 7.83(4H,bs), 10.33(1H,s)

enzyme inhibiting activity IC50 of trypsin 4.8 × 10-7
of thrombin 7.0 × 10-8

[Example 95]

At ice bath temperature, 3.30g of 4-guanidinobenzoylchloride hydrochloride was added to a mixture of 4.40g of N- phenylpiperazinylcarbonylmethyl 2-(4-hydroxyphenyl)propionate and 30ml of pyridine. After warming the mixture slowly up to room temperature, it was stirred overnight, and concentrated under reduced pressure. Ether was added to the residue by decantation, and the mixture was dissolved in 50ml of water. In an ice bath, a solution of 3.59g of potassium hydrogencarbonate in 50ml of water was added dropwise to the mixture. After the mixture was stirred for 3 hours in an ice bath, the deposit crystals were collected by filteration, washed successively with water and acetone, and 4.98g of carbonate was collected. In an ice bath, 0.62g of methane sulfonic acid was added to a solution of 2.00g of the carbonate in

methanol. After some insoluble substances were filtered, and concentrated under reduced pressure. After aceton was added to the residue, the mixture was azeotropic boiled, and 2.69g of foaminess crystals were collected. 0.96g of the crystals were crystallized from dichlorlmethane-IPE. The yield of 2-[4-(4-guanidinobenzoyloxy)phenyl]propionic acid N-phenylpiperazinylcarbonylmethylester • 2 methane sulfonate was 0.76g. This compound had 3mol of water and 1/3mol of dichloromethane by NMR and didn't show clear melting point.

NMR (DMSO-d6) δ

1.50(3H,d), 2.50(6H,s), 3.37(4H,m),
3.70(4H,m), 3.83(1H,q), 4.90(2H,s),
5.40(6H,bs), 5.67(2/3H,s), 7.1-7.6(11H,m),
7.83(5H,bs), 8.13(2H,d), 10.30(1H,s)

enzyme inhibiting activity IC50 of trypsin 3.8 × 10-7
of thrombin 4.7 × 10-8

[Example 96]

At ice bath temperature, 5.00g of 4-guanidinobenzoylchloride hydrochloride was added to a mixture of 3.50g of (S)-(+)-2-(4hydroxyphenyl)propionic acid methylester($[\alpha]_D^{20}$ = +91.8° (c=1.00, EtOH)) and 30ml of pyridine. After warming the mixture slowly up to room temperature, it was stirred overnight, and concentrated under reduced pressure. Ether was added to the residue by decantation, and the mixture was dissolved in 100ml of water. In an ice bath, a solution of 5.83g of potassium hydrogencarbonate in 30ml of water was added dropwise to the mixture. After the mixture was stirred for 3 hours at the same temperature, the deposit crystals were collected by filteration, washed successivly with water and acetone, and 6.97g of carbonate was collected. In an ice bath, 1.69g of methane sulfonic acid was added to a solution of 6.75g of the carbonate in acetone, some insolubles were filtered, and concentrated under reduced pressure. Ether was added to the residue, the deposit crystals were collected by filteration, and washed with ether. The yield of (S)-(+)-2-[4-(4- guanidinobenzoyloxy) phenyl] propionic acid methylester • methane sufonate was 7.06g.

m.p. 166.5-170°C
= +31.7° (c=1.00, H2O)
NMR (DMSO-d6) δ

1.43(3H,d), 2.50(3H,s), 3.58(3H,s),
3.80(1H,q), 7.14(2H,d), 7.33(2H,d),
7.37(2H,d), 7.76(4H,bs),
8.14(2H,d), 10.19(1H,s)

enzyme inhibiting activity IC50 of trypsin 3.0 × 10-7

[Example 97]

At ice bath temperature, 5.00g of 4-guanidinobenzoylchloride hydrochloride was added to a mixture of 4.04g of (S)-(+)-2-(4-hydroxyphenyl)propionic acid isopropylester($[\alpha]_D^{20}$ = +46.7° (c=1.00, EtOH)) and 30ml of pyridine. After warming the mixture slowly up to room temperature, it was stirred overnight, and concentrated under reduced pressure. Ether was added to the residue by decantation, and the mixture was dissolved in 150ml of water. In an ice bath, a solution of 5.83g of potassium hydrogencarbonate in 150ml of water was added dropwise to the mixture. After the mixture was stirred for 5 hours at the same temperature, the mixture was let stand overnight in a refrigerator. The deposit crystals were collected by filteration, washed suc cessivly with water and acetone, and 7.57g of carbonate was collected. After 1.75g of methane sulfonic acid was added to a solution of 7.50g of carbonate in acetone, some insoluble substances were collected by filteration, and concentrated under reduced pressure. Acetone and ether were added to the residue, the deposit crystals were collected by filteration, and washed with ether. The yield of (S)-(+)-2-[4-(4-guanidinobenzoyloxy) phenyl] propionic acid isopropylester • methane sufonate was 7.82g.

mp 111-113°C
$[\alpha]_D^{20}$ = +13.1° (c=1.00, H2O)
NMR (DMSO-d6) δ

1.13(3H,d), 1.20(3H,d), 1.43(3H,d),
2.50(3H,s), 3.76(1H,q), 4.89(1H,quint),
7.16(2H,d), 7.36(2H,d), 7.39(2H,d),
7.77(4H,bs), 8.13(2H,d), 10.21(1H,s)

enzyme inhibiting activity IC50 of trypsin 3.6 × 10-7

[Example 98]

At ice bath temperature, 4.02g of 4-guanidinobenzoylchloride hydrochloride was added to a mixture of 4.00g of (S)-(+)-2-(4hydroxyphenyl)propionic acid benzylester($[\alpha]_D^{20}$ = +25.6° (c=1.00, EtOH)) and 30ml of pyridine. After warming the mixture slowly up to room temperature, it was stirred overnight, and concentrated under reduced pressure. Ether was added to the residue by decantation, and the mixture was dissolved in 150ml of water. In an ice bath, a solution of 4.69g of potassium hydrogencarbonate in 30ml of water was added dropwise to the mixture. After the mixture was stirred for 4 hours at the same temperature, the deposit crystals were collected by filteration. These crystals were dis solved in 50ml of water, the deposit crystals were collected by filteration, washed successivly with water and acetone, and 6.15g of carbonate was collected. After 0.63g of methane sulfonic acid was added to a solution of 3.00g of carbonate in acetone. After some insoluble substances were filtered, it was concentrated under reduced pressure. Ether was added to the residue, the deposit crytstals were collected by filteration, and they were washed with ether. The yield of (S)-(+)-2-[4-(4- guanidino benzoyloxy) phenyl] propionic acid benzylester • methane sufonate was 3.15g.
m.p. 129-131°C
$[\alpha]_D^{20}$ = +3.3° (c=1.00,H2O)
NMR (DMSO-d6) δ
1.47(3H,d), 2.52(3H,s), 3.91(1H,q),
5.10(2H,s), 7.26(5H,bs), 7.41(2H,d),
7.79(4H,bs), 8.15(2H,d), 10.20(1H,s)
enzyme inhibiting activity IC50 of trypsin 3.05 × 10-7

[Example 99]

(R)-(-)-2-[4-(4-guanidinobenzoyloxy)phenyl]propionic acid benzylester • methane sulfonate was prepared in the same procedure described at [Example 98] except for using (R)-(-)-2-(4-hydroxyphenyl)-propionic acid benzylester($[\alpha]_D^{20}$ = -25.4° (c=1.00, EtOH)) as a starting material. m.p. 126-129°C
$[\alpha]_D^{20}$ = -4.6° (c=1.00, H2O)
NMR (DMSO-d6) δ
1.47(3H,d), 2.52(3H,s), 3.90(1H,q),
5.08(2H,s), 7.26(5H,bs), 7.39(2H,d)
7.77(4H,bs), 8.12(2H,d), 10.19(1H,s)
enzyme inhibiting activity IC50 of trypsin 3.75 × 10-7

[Example 100]

At ice bath temperature, 3.25g of 4-guanidinobenzoylchloride hydrochloride was added to a mixture of 3.13g of (S)-(+)-2-(4hydroxyphenyl)propionic acid 2,2,2-trifluoroethyl ester($[\alpha]_D^{20}$ = +57.7° (c=1.00, EtOH)) and 30ml of pyridine. After warming the mixture slowly up to room temperature, it was stirred overnight, and concentrated under reduced pressure. Ether was added to the residue by decantation, and the mixture was dissolved in 150ml of water. In an ice bath, a solution of 3.79g of potassium hydrogencarbonate in 30ml of water was added dropwise to the mixture. After the mixture was stirred for 8 hours at the same temperature, the mixture was let stand overnight in a refrigerator. The deposit crystals were collected by filteration, washed successively with water and acetone, and 5.34g of carbonate was collected. In an ice bath, 1.06g of methane sulfonic acid was added to a solution of 4.96g of the carbonate in methanol. After some insoluble substances were filtered, the deposit crystals were collected by filteration, and concentrated under reduced pressure. Ether was added to the residue, the deposit crystals were collected by filteration, and they were washed with ether. The yield of (S)-(+)-2-[4-(4-guanidinobenzoyloxy)phenyl]propionic acid 2,2,2-trifluoroethyl ester • methane sufonate was 4.57g.
m.p. 123-126°C
$[\alpha]_D^{20}$ = +17.0° (c=1.00, H2O)
NMR (DMSO-d6) δ
1.50(3H,d), 2.58(3H,s), 3.95(1H,q),
4.69(2H,q), 7.20(2H,d), 7.42(2H,d)
7.44(2H,d), 7.84(4H,bs), 8.17(2H,d)

10.06(1H,s)
enzyme inhibiting activity IC50 of trypsin 3.5 × 10-7

[Example 101]

At ice bath temperature, 5.00g of 4-guanidinobenzoylchloride hydrochloride was added to a mixture of 4.33g of (S)-(+)-2-(4hydroxyphenyl)propionic acid carbamoylmethylester ($[\alpha]_D^{20}$ = +83.4° (c=1.00, EtOH)) and 30ml of pyridine. After warming the mixture slowly up to room temperature, it was stirred overnight, and concentrated under reduced pressure. Ether was added to the residue by decantation, and the mixture was dissolved in 100ml of water. In an ice bath, a solution of 5.83g of potassium hydrogencar bonate in 30ml of water was added dropwise to the mixture. After the mixture was stirred for 5.5 hours at the same temperature, the mixture was let stand overnight in a refrigerator. The deposit crystals were collected by filteration, washed successively with water and acetone, and 7.65g of carbonate was collected. In an ice bath, 1.71g of methane sulfonic acid was added to a solution of 7.56g of the carbonate in methanol. After some insoluble substances were filtered, the mixture was concentrated under reduced pressure. Acetone was added to the residue, the deposit crystals were collected by filteration, and they were washed with acetone. The yield of (S)-(+)-2-[4-(4- guanidinobenzoyloxy) phenyl] propionic acid carbamoylmethylester • methane sufonate was 7.76g.
m.p. 174-177°C
$[\alpha]_D^{20}$ = +34.7° (c=1.00, H2O)
NMR (DMSO-d6) δ
1.47(3H,d), 2.56(3H,s), 3.95(1H,q),
4.40(1H,d), 4.43(1H,d), 6.8-7.6(2H,br)
7.20(2H,d), 7.41(2H,d), 7.43(2H,d)
7.81(4H,bs), 8.18(2h,d), 10.21(1H,s)
enzyme inhibiting activity IC50 of trypsin 3.8 × 10-7

[Example 102]

At ice bath temperature, 3.59g of 4-guanidinobenzoylchloride hydrochloride was added to a mixture of 3.50g of (S)-(+)-2-(4hydroxyphenyl)propionic acid N,N-dimethylcarbamoylmethyl ester ($[\alpha]_D^{20}$ = +80.9° (c=1.00, EtOH)) and 30ml of pyridine. After warming the mixture slowly up to room temperature, it was stirred over night, and concentrated under reduced pressure. Ether was added to the residue by decantation, and the mixture was dissolved in 100ml of water. In an ice bath, a solution of 4.18g of potassium hydrogencarbonate in 30ml of water was added dropwise to the mixture. After the mixture was stirred for 6.5 hours at the same temperature, the mixture was let stand overnight in a refrigerator. The deposit crystals were collected by filteration, washed successively with water and acetone, and 5.39g of carbonate was collected. In an ice bath, 1.14g of methane sulfonic acid was added to a solution of 5.37g fo the carbonate in methanol. After some insoluble substance were filtered, the filterate was concentrated under reduced pressure. Acetone was added to the residue, the deposit crystals were collected by filteration, and they were washed with acetone. The yield of (S)- (+)-2-[4-(4-guanidino benzoyloxy) phenyl] propionic acid N,N- dimethylcarbamoyl methylester • methane sufonate was 3.57g. This compound had 1mol of water by NMR.
m.p. 102-104°C
$[\alpha]_D^{20}$ = + 39.3° (c=1.00, H2O)
NMR (DMSO-d6) δ
1.47(3H,d), 2.47(3H,s), 2.82(3H,s),
2.88(3H,s), 3.47(2H,bs), 3.93(1H,q)
4.76(2H,s), 7.20(2H,d), 7.43(2H,d)
7.44(2H,d), 7.80(4H,bs), 8.16(2H,d)
10.22(1H,s)
enzyme inhibiting activity IC50 of trypsin 2.4 × 10-7

[Example 103]

(R)-(-)-2-[4-(4-guanidinobenzoyloxy)phenyl]propionic acid N,N-dimethylcarbamoylmethyl ester • methane sulfonate was prepared in the same procedure described at [Example 102] except for using (R)-(-)-2-(-hydroxyphenyl)propionic acid N,N-dimethylcarbamoyl methyl ester($[\alpha]_D^{20}$ = -80.2° (c=1.00, EtOH)) as a

starting material.
m.p. 102-105°C
$[\alpha]_D^{20}$ = -39.0° (c=1.00, H2O)
NMR (DMSO-D6) δ
1.47(3H,d), 2.50(3H,s), 2.84(3H,s),
2.89(3H,s), 3.50(2H,bs), 3.92(1H,q)
4.76(2H,s), 7.19(2H,d), 7.41(2H,d)
7.44(2H,d), 7.79(4H,bs), 8.14(2H,d),
10.23(1H,s)
enzyme inhibiting activity IC50 of trypsin 2.65 × 10-7

[Example 104]

At ice bath temperature, 5.00g of 4-guanidinobenzoylchloride hydrochloride was added to a mixture of 5.70g of (S)-(+)-2-(4-hydroxyphenyl)propionic acid morpholinocarbonylmethyl ester($[\alpha]_D^{20}$ = +68.3° (c=1.00, EtOH)) and 30ml of pyridine. After warming the mixture slowly up to room temperature, it was stirred overnight, and concentrated under reduced pressure. Ether was added to the residue by decantation, and the mixture was dissolved in 150ml of water. In an ice bath, a solution of 5.83g of potassium hydrogencarbonate in 30ml of water was added dropwise to the mixture. After the mixture was stirred for 7 hours at the same temperature, the deposit crystals were collected by filteration. They were washed successively with water and acetone, and 5.01g of carbonate was collected. In an ice bath, 0.75g of methane sulfonic acid was added to a solution of 3.91g of the carbonate in methanol. After some insoluble substances were filtered, the mixture was concentrated under reduced pressure. Acetone was added to the residue, the mixture was concentrated under reduced pressure again. Ether was added, the deposit crystals were collected by filteration, and washed with ether. The yield of (S)-(+)-2-[4-(4- guanidinobenzoyloxy) phenyl] propionic acid morpholinocarbonyl methylester • methane sufonate was 3.85g.
m.p. 133-136°C
$[\alpha]_D^{20}$ = +39.4° (c=1.00, H2O)
NMR (DMSO-d6) δ
1.48(3H,d), 2.51(3H,s), 3.2-3.8(8H,m),
3.94(1H,q), 4.76(2H,s), 7.14(2H,d)
7.36(2H,d), 7.37(2H,d), 7.74(4H,bs)
8.09(2H,d), 10.16(1H,s)
enzyme inhibiting activity IC50 of trypsin 3.4 × 10-7

[Example 105]

At ice bath temperature, 5.00g of 4-guanidinobenzoylchloride hydrochloride was added to a mixture of 5.31g of (S)-(+)-2-(4-hydroxyphenyl)propionic acid 4-fluorobenzyl ester ($[\alpha]_D^{20}$ = +25.6° (c=1.00, EtOH)) and 30ml of pyridine. After warming the mixture slowly up to room temperature, it was stirred overnight, and concentrated under reduced pressure. Ether was added to the residue by decantation, and the mixture was dissolved in 150ml of water. In an ice bath, a solution of 5.83g of potassium hydrogencarbonate in 50ml of water was added dropwise to the mixture. After the mixture was stirred for 5.5 hours at the same temperature, the mixture was let stand overnight in a refrigerator. The deposit crystals were collected by filteration, washed successively with water and acetone, and 8.23g of carbonate was collected. In an ice bath, 1.67g of methane sulfonic acid was added to a solution of 8.21g of the carbonate in actone. After some insoluble substances were filtered, the mixture was concentrated under reduced pressure. Ether was added to the residue, the deposit crystals were collected by filteration, and they were washed with ether. The yield of (S)-(+)-2-[4-(4- guanidinobenzoyloxy) phenyl] propionic acid 4-fluorobenzylester • methane sufonate was 8.79g.
m.p. 125-128°C
$[\alpha]_D^{20}$ = + 4.5° (c=1.00, H2O)
NMR (DMSO-d6) δ
1.46(3H,d), 2.51(3H,s), 3.89(1H,q),
5.08(2H,s), 6.9-7.6(6H,m), 7.80(4H,bs)
8.14(2H,d), 10.28(1H,s)
enzyme inhibiting activity IC50 of trypsin 5.4 × 10-7

[Example 106]

At ice bath temperature, 5.00g of 4-guanidinobenzoylchloride hydrochloride was added to a mixture of 4.63g of (S)-(+)-2-(4-hydroxyphenyl)propionic acid methoxycarbonylmethylester ($[\alpha]_D^{20}$ = + 76.4° (c=1.00, EtOH)) and 30ml of pyridine. After warming the mixture slowly up to room temperature, it was stirred overnight, and concentrated under reduced pressure. Ether was added to the residue by decantation, and the mixture was dissolved in 150ml of water. In an ice bath, a solution of 5.83g of potassium hydrogencarbonate in 30ml of water was added dropwise to the mixture. After the mixture was stirred for 8 hours at the same temperature, the mixture was let stand overnight in a refrigerator. The deposit crystals were collected by filteration, washed successively with water and acetone, and 8.24g of carbonate was collected. In an ice bath, 1.77g of methane sulfonic acid was added to a solution of 8.10g of the carbanate in acetone, some in soluble substances were filtered, and concentrated under reduced pressure. Ether was added to the residue, the deposit crystals were collected by filteration, and washed with ether. The yield of (S)-(+)-2-[4-(4-guanidinobenzoyloxy)phenyl]propionic acid methoxycarbonylmethyl ester • methane sufonate was 8.50g.
m.p. 81-83°C
$[\alpha]_D^{20}$ = + 38.7° (c=1.00, H2O)
NMR (DMSO-d6) δ
1.47(3H,d), 2.51(3H,s), 3.62(3H,s),
3.95(1H,q), 4.65(2H,s), 7.16(2H,d)
7.38(2H,d), 7.40(2H,d), 7.78(4H,bs),
8.14(2H,d), 10.21(1H,s)
enzyme inhibiting activity IC50 of trypsin 2.7 × 10-7

[Example 107]

At ice bath temperature, 3.57g of 4-guanidinobenzoylchloride hydrochloride was added to a mixture of 3.50g of (S)-(+)-2-(4-hydroxyphenyl)propionic acid ethoxycarbonylmethylester ($[\alpha]_D^{20}$ = + 71.3° (c=1.00, EtOH)) and 30ml of pyridine. After warming the mixture slowly up to room temperature, it was stirred overnight, and concentrated under reduced pressure. Ether was added to the residue by decantation, and the mixture was dissolved in 150ml of water. In an ice bath, a solution of 4.17g of potassium hydrogencarbonate in 30ml of water was added dropwise to the mixture. After the mixture was stirred for 4 hours at the same temperature, the deposit crystals were collected by filteration. They were washed successively with water and acetone, and 5.65g of carbonate was collected. In an ice bath, 0.42g of methane sulfonic acid was added to a solution of 5.51g of the carbonate in acetone. After some insoluble substances were filtered, and concentrated under reduced pressure. Ether was added to the residue, the deposit crystals were collected by filteration, and they were washed with ether. The yield of (S)-(+)-2-[4-(4-guanidinobenzoyloxy)-phenyl]propionic acid ethoxycarbonyl methylester • methane sufonate was 5.36g.
m.p. 115-118°C
$[\alpha]_D^{20}$ = + 35.6° (c=1.00, H2O)
NMR (DMSO-d6) δ
1.22(3H,t), 1.49(3H,d), 2.50(3H,s),
3.97(1H,q), 4.13(2H,q), 4.67(2H,s),
7.23(2H,d), 7.44(2H,d), 7.46(2H,d),
7.84(4H,bs), 8.19(2H,d), 10.27(1H,s)
enzyme inhibiting activity IC50 of trypsin 2.6 × 10-7

[Example 108]

(R)-(-)-2-[4-(4-guanidinobenzoyloxy)phenyl]propionic acid ethoxycarbonylmethylester • methane sulfonate was obtained from the same procedure described at [Example 107] except for using (R)-(-)-2-(-hydroxyphenyl)propionic acid ethoxycarbonylmethyl ester($[\alpha]_D^{20}$ = -71.6° (c=1.00, EtOH)) as a starting material.
m.p. 115-117°C
$[\alpha]_D^{20}$ = -36.5° (c=1.00, H2O)
NMR (DMSO-d6) δ
1.18(3H,t), 1.48(3H,d), 2.47(3H,s),
3.93(1H,q), 4.10(2H,q), 4.61(2H,s)

7.16(2H,d), 7.37(2H,d), 7.41(2H,d)
7.76(4H,bs), 8.11(2H,d), 10.17(1H,s)
enzyme inhibiting activity IC50 of trypsin 2.6 × 10-7

[Example 109]

At ice bath temperature, 5.00g of 4-guanidinobenzoylchloride hydrochloride was added to a mixture of 5.44g of (S)-(+)-2-(4hydroxyphenyl)propionic acid t-buthoxycarbonylmethylester ($[\alpha]_D^{20}$ = + 63.2° (c=1.00, EtOH)) and 30ml of pyridine. After warming the mixture slowly up to room temperature, it was stirred overnight, and concentrated under reduced pressure. Ether was added to the residue by decantation, and the mixture was dissolved in 100ml of water. In an ice bath, a solution of 5.83g of potassium hydrogencarbonate in 30ml of water was added dropwise to the mixture. After the mixture was stirred for 4 hours at the same temperature, the deposit crystals were collected by filteration. They were washed successively with water and acetone, and 9.60g of carbonate was collected. In an ice bath, 1.92g of methane sulfonic acid was added to a solution of 9.60g of the carbonate in acetone. After some insoluble substances were filtered, and concentrated under reduced pressure. Acetone and ether were added to the residue, the deposit crystals were collected by filteration, and they were washed with ether. The yield of (S)-(+)-2-[4-(4-guanidinobenzoyloxy) phenyl] propionic acid t-buthoxy- carbonylmethylester • methane sufonate was 8.17g.
m.p. 103-106°C
$[\alpha]_D^{20}$ = + 33.5° (c=1.00, H2O)
NMR (DMSO-d6) δ
1.39(9H,s), 1.48(3H,d), 2.56(3H,s),
3.88(1H,q), 4.46(2H,s), 7.13(2H,d),
7.36(4H,d), 7.72(4H,bs), 8.06(2H,d),
10.36(1H,s)
enzyme inhibiting activity IC50 of trypsin 3.0 × 10-7

[Example 110]

At ice bath temperature, 15.00g of 4-guanidinobenzoyl chloride hydrochloride was added to a mixture of 18.31g of (S)-(+)-2-(4-hydroxyphenyl)propionic acid benzyloxycarbonylmethyl ester($[\alpha]_D^{20}$ = + 61.5° (c=1.00, EtOH)) and 60ml of pyridine. After warming the mixture slowly up to room temperature, it was stirred overnight, and concentrated under reduced pressure. Ether was added to the residue by decantation, and the mixture was dis solved in 250ml of water. In an ice bath, a solution of 17.50g of potassium hydrogencarbonate in 100ml of water was added dropwise to the mixture. After the mixture was stirred for 3.5 hours at the same temperature, the deposit crystals were collected by filteration. They were washed successively with water and acetone, and 25.27g of carbonate was collected. In an ice bath, 1.90g of methane sulfonic acid was added to a soluiton of 10.12g of the carbonate in acetone. After some insoluble substances were filtered, the mixture was concentrated under reduced pressure. Ether was added to the residue, the deposit crystals were collected by filteration, and they were washed with ether. The yield of (S)-(+)-2-[4-(4-guanidinobenzoyloxy)phenyl]propionic acid benzyloxycarbonylmethyl ester • methane sufonate was 9.79g.
m.p. 143-146°C
$[\alpha]_D^{20}$ = + 31.5° (c=1.00, H2O)
NMR (DMSO-d6) δ
1.47(3H,d), 2.51(3H,s), 3.93(1H,q),
4.69(2H,s), 5.12(2H,s), 7.15(2H,d),
7.31(5H,s), 7.38(2H,d), 7.47(2H,d),
7.79(4H,bs), 8.13(2H,d), 10.21(1H,s)
enzyme inhibiting activity IC50 of trypsin 3.9 × 10-7

[Example 111]

A solution of 15.00g of a carbonate of (S)-(+)-2-[4-(4- guanidinobenzoyloxy) phenyl] propionic acid benzyloxycarbonyl methylesther which was obtained from [Example 110] in 300ml of acetic acid was hydrogenated in the presence of 1.50g of 10% Pb- C(containing 48.3% of water) at ordinary temperature and pressure. The catalyst was filtered, concentrated under reduced pressure, and ether was added to the

residue. The deposit crystals were collected by filteration, washed with ether, and 10.63g of crystals were collected. They were dissolved in 50ml of water, adjusted to pH8 with saturated sodium hydrogencarbonate solution. The deposit crystals were collected by filteration, and washed successively with water and acetone. In an ice bath, 2.66g of methane sulfornic acid was added to a solution of the craystals in 50ml of methanol. Some insoluble substances were filtered, concentrated under reduced pressure, and ether was added. The deposit crystals were collected by filteration, and washed with ether. The yield of (S)-(+)-2-[2-[4-(4- guanidinobenzoyloxy) phenyl] propionyloxy]acetic acid • methane sulfonate was 12.30g.

m.p. 112-115 °C

$[\alpha]_D^{20}$ = + 34.0° (c = 1.00, H2O)

NMR (DMSO-d6) δ

1.47(3H,d), 2.49(3H,s), 3.94(1H,q),
4.54(2H,s), 7.17(2H,d), 7.41(2H,d),
7.72(2H,d), 7.78(5H,bs), 8.16(2H,d),
10.21(1H,s)

enzyme inhibiting activity IC50 of trypsin $3.4 \times 10^{-7}$7

[Example 112]

A solution of 3.10g of a carbonate of (S)-(+)-2-[4-(4- guanidinobenzoyloxy)phenyl]propionic acid benzylesther which was obtained from [Example 98] in 62ml of acetic acid was hydrogenated in the presence of 0.31 g of 10% Pb-C(containing 48.3% of water) at ordinary temperature and pressure. The catalyst was filtered, concentrated under reduced pressure, and ether was added to the residue. The deposit crystals were collected by filteration, washed with ether, and 2.44g of crystals were collected. They were dissolved in 24ml of water. At ice bath temperature, adjusted to pH8 with saturated sodium hydrogencar bonate solution, and stirred for 1 hour at the same temperature. The deposit crystals were collected by filteration, washed successively with water and acetone, and 2.06g of crystals were collected. In all ice bath, 0.64g of methane sulfornic acid was added to a solution of the crystals in methanol. Some insoluble substances were filtered, and concentrated under reduced pressure. Ether was added to the residue, and concentrated under reduced pressure. Ether was added, deposit crystals were collected by filteration, and washed with ether. The yield of (S)-(+)-2-[4-(4-guanidino benzoyloxy) phenyl] propionic acid • methane sulfonate was 2.53g.

m.p. 150-153 °C

$[\alpha]_D^{20}$ = + 42.3° (c = 1.00, H2O)

NMR (DMSO-d6) δ

1.47(3H,d), 2.50(3H,s), 3.70(1H,q),
7.14(2H,d), 7.37(2H,d), 7.38(2H,d),
7.75(4H,bs), 8.10(2H,d), 10.17(1H,s)

enzyme inhibiting activity IC50 of trypsin $2.8 \times 10^{-7}$7

[Example 113]

(R)-(-)-2-[4-(4-guanidinobenzoyloxy)phenyl]propionic acid • methanesulfonate was prepared in the same procedure described at [Example 112] except for using a carbonate of (R)-(-)-2-[4-(4guanidinobenzoyloxy)-phenyl]propionic acid benzylester which was prepared from [Example 99] as a starting material.

m.p. 149-151 °C

$[\alpha]_D^{20}$ = -43.5° (c = 1.00, H2O)

NMR (DMSO-d6) δ

1.41(3H,d), 2.50(3H,s), 3.70(1H,q),
7.10(2H,d), 7.33(2H,d), 7.34(2H,d),
7.73(4H,bs), 8.07(2H,d), 10.13(1H,s)

enzyme inhibiting activity IC50 of trypsin $2.8 \times 10^{-7}$7

[Exapmle 114]

At ice bath temperature, 5.00g of 4-guanidinobenzoylchloride hydrochloride was added to a mixture of 5.38g of (S)-(+)-2-(4hydroxyphenyl)propionic acid pyrrolidinocarbonylmethyl ester ($[\alpha]_D^{20}$ = + 75.0° (c = 1.00, EtOH)) and 30ml of pyridine. After warming the mixture slowly up to room temperature, it was stirred overnight, and the reaction mixture was concentrated under reduced pressure. The ether was added

to the residue by decantation, and then 150ml of water was added. At ice bath temperature, a solution of 5.83g of potassium hydrogencarbonate in 30ml of water was added dropwise to the mixture. After the mixture was stirred for 4 hours at the same temperature, it was let stand overnight in a refrigerator. The deposit crystals were collected by filteration, washed successively with water and acetone, and 8.55g of a carbonate was collected. At ice bath temperature, 1.71g of methane sulfonic acid was added to a s olution of 8.48g of the carbonate in methanol. Some insoluble substances were filtered, concentrated under reduced pressure, and ether was added to the residue. The deposit crystals were collected by filteration, and washed with ether. The yield of (S)-(+)-2-[4-(4- guanidinobenzoyloxy) phenyl] propionic acid pyrrolidinocarbonyl methylester • methane sulfonate was 8.85g.

m.p. 174-176°C

$[\alpha]_D^{20}$ = + 41.1° (c=1.00, H2O)

NMR (DMSO-d6) δ

1.47(3H,d), 1.6-2.2(4H,m), 2.50(3H,s),
3.21(4H,bt), 3.90(1H,q), 4.64(2H,s),
7.14(2H,d), 7.37(2H,d), 7.43(2H,d),
7.77(4H,bs), 8.11(2H,d), 10.19(1H,s)

enzyme inhibiting activity IC50 of trypsin $2.8\times10^{-7}$ 7

[Exapmle 115]

At ice bath temperature, 4.01g of 4-guanidinobenzoylchloride hydrochloride was added to a mixture of 5.10g of (S)-(+)-2-(4hydroxyphenyl)propionic acid N-benzyl-N-methylcarbamoyl methyl ester($[\alpha]_D^{20}$ = + 67.8° (c=1.00, EtOH)) and 30ml of pyridine. After warming the mixture slowly up to room temperature, it was stirred overnight, and the reaction mixture was concentrated under reduced pressure. Ether was added to the residue by decantation, and then 150ml of water was added. At ice bath temperature, a solution of 4.68g of potassium hydrogencarbonate in 30ml of water was added dropwise to the mixture. The crystals was dispersed. After the mixture was stirred for 3 hours at the same temperature, it was let stand overnight in a refrigerator. The deposit crystals were collected by filteration, washed successively with water and acetone, and 2.90g of carbonate was collected. At ice bath temperature, 0.46g of methane sulfonic acid was added to a solution of 2.50g of the carbonate in methanol. Some insoluble substances were filtered, concentrated under reduced pressure, and ether was added to the residue. The deposit crystals were collected by filtration, and washed with ether. The yield of (S)-(+)-2-[4-(4- guanidinobenzoyloxy) phenyl] propionic acid N-benzyl-N- methylcarbamoylmethylester • methane sulfonate was 2.45g.

m.p. 129-132°C

$[\alpha]_D^{20}$ = + 32.3° (c=1.00, H2O)

NMR (DMSO-d6) δ

1.50(3H,d), 2.52(3H,s), 2.86(3H,s),
3.94(1H,q), 4.51(2H,s), 4.89(2H,s),
7.21(2H,d), 7.27(5H,s), 7.45(2H,d),
7.46(2H,d), 7.79(4H,bs), 8.16(2H,d),
10.21(1H,s)

enzyme inhibiting activity IC50 of trypsin $3.1\times10^{-7}$7

[Exapmle 116]

At ice bath temperature, 5.00g of 4-guanidinobenzoylchloride hydrochloride was added to a mixture of 3.77g of (S)-(+)-2-(4hydroxyphenyl)propionic acid ethylester($[\alpha]_D^{20}$ = +65.2°K(c=1.00, EtOH)) and 30ml of pyridine. After warming the mixture slowly up to room temperature, it was stirred overnight, and the reaction mixture was concentrated under reduced pressure. Ether was added to the residue by decantation, and then 100ml of water was added. At ice bath temperature, a solution of 5.83g of potassium hydrogen carbonate in 30ml of water was added dropwise to the mixture. After the mixture was stirred for 4 hours at the same temperature, it was let stand overnight in a refrigerator. The deposit crystals were collected by filteration, washed successively with water and acetone, and 7.12g of carbonate was collected. At ice bath temperature, 1.69g of methane sulfonic acid was added to a solution of 7.00g of carbonate in acetone. Some insoluble substances were filtered, concentrated under reduced pressure, and acetone was added to the residue. The deposit crystals were collected by filteration, and washed with acetone. The yield of (S)-(+)-2-[4-(4-guanidinobenzoyloxy) phenyl] propionic acid ethylester • methane sulfonate was 4.77g.

m.p. 134-136°C

$[\alpha]_D^{20}$ = + 21.6° (c=1.00, H2O)
NMR (DMSO-d6) δ
1.16(3H,t), 1.44(3H,d), 2.48(3H,s),
3.82(1H,q), 4.07(2H,q), 7.06(2H,d),
7.40(2H,d), 7.43(2H,d), 7.80(4H,bs),
8.16(2H,d), 10.19(1H,s)
enzyme inhibiting activity IC50 of trypsin $2.2\times10^{-7}$7

[Example 117]

(R)-(-)-2-[4-(4-guanidinobenzoyloxy)phenyl]propionic acid ethylester • methane sulfonate was prepared in the same procedure described at [Example 116] except for using a carbonate of (R)-(-)-2-(4-hydroxyphenyl) propionic acid ethylester($[\alpha]_D^{20}$ = -66.0° (c=1.00, EtOH)) as a starting material.
m.p. 133-135°C
$[\alpha]_D^{20}$ = -21.9° (c=1.00, H2O)
NMR (DMSO-d6) δ
1.16(3H,t), 1.42(d,3H), 2.50(3H,s),
3.78(1H,q), 4.06(2H,q), 7.17(2H,d),
7.37(2H,d), 7.41(2H,d), 7.79(4H,bs),
8.12(2H,d), 10.21(1H,s),
enzyme inhibiting activity IC50 of trypsin 2.3 × 10-7

[Exapmle 118]

At ice bath temperature, 5.00g of 4-guanidinobenzoylchloride hydrochloride was added to a mixture of 5.42g of (S)-(+)-2-(4hydroxyphenyl)propionic acid N,N-diethylcarbamoyl methyl ester ($[\alpha]_D^{20}$ = + 76.9° (c=1.00, EtOH)) and 30ml of pyridine. After warming the mixture slowly up to room temperature, it was stirred overnight, and the reaction mixture was concentrated under reduced pressure. Ether was added to the residue by decantation, and then 100ml of water was added. At ice bath temperature, a solution of 5.83g of potassium hydrogencarbonate in 30ml of water was added dropwise to the mixture. After the mixture was stirred for 2.5 hours at the same temperature, it was let stand overnight in a refrigerator. The deposit crystals were collected by filtration, washed successively with water and acetone, and 8.30g of carbonate was collected. At ice bath temperature, 1.62g of methane sulfonic acid was added to a solution of 8.06g of the carbonate in acetone. Some insoluble substances were filtered, and the filterate was concentrated under reduced pressure. After acetone was added to the residue, the deposit crystals were collected by filteration, and they were washed with acetone. The yield of (S)-(+)-2-[4-(4-guanidinobenzoyloxy)phenyl]propionic acid N,N-diethylcarbamoylmethyl ester • methane sulfonate was 8.07g.
m.p. 122-126°C
$[\alpha]_D^{20}$ = + 40.1° (c=1.00, H2O)
NMR (DMSO-d6) δ
1.08(6H,bt), 1.50(3H,d), 2.50(3H,s),
3.24(4H,bq), 3.92(1H,q), 4.73(2H,s),
7.17(2H,d), 7.40(2H,d), 7.43(2H,d),
7.77(4H,bs), 8.14(2H,d), 10.19(1H,s),
enzyme inhibiting activity IC50 of trypsin 2.8 × 10-7

[Exapmle 119]

At ice bath temperature, 5.00g of 4-guanidinobenzoylchloride hydrochloride was added to a mixture of 5.66g of (S)-(+)-2-(4hydroxyphenyl) propionic acid piperidinocarbonylmethylester ($[\alpha]_D^{20}$ = + 69.2° (c=1.00, EtOH)) and 30ml of pyridine. After warming the mixture slowly up to room temperature, it was stirred overnight, and the reaction mixture was concentrated under reduced pressure. Ether was added to the residue by decantation, and then 100ml of water was added. At ice bath temperature, a solution of 5.83g of potassium hydrogen carbonate in 30ml of water was added dropwise to the mixture. After the mixture was stirred for 3 hours at the same temperature, the deposit crystals were collected by filteration. They were washed successively with water and acetone, and 6.80g of carbonate was collected. At ice bath temperature, 1.32g of methane sulfonic acid was added to a solution of 6.75g of the carbonate in methanol. Some insoluble substances were filtered, and concentrated under reduced pressure. Acetone was added to

the residue, and concentrated under reduced pressure again. After acetone was added to the residue, the deposit crystals were collected by filteration, and they were washed with acetone. The yield of (S)-(+)-2-[4-(4-guanidinobenzoyloxy) phenyl] propionic acid piperidinocarbonyl methyl ester • methane sulfonate was 6.98g.

m.p. 106-109°C

$[\alpha]_D^{20}$ = + 39.4° (c=1.00, H2O)

NMR (DMSO-d6) δ

1.49(3H,d), 1.55(6H,bs), 2.52(3H,s),
3.34(4H,bs), 3.93(1H,q), 4.77(2H,s),
7.20(2H,d), 7.45(2H,d), 7.47(2H,d),
7.78(4H,bs), 8.18(2H,d), 10.20(1H,s),

enzyme inhibiting activity IC50 of trypsin 2.8 × 10-7

[Exapmle 120]

At ice bath temperature, 1.79g of 4-guanidinobenzoylchloride hydrochloride was added to a mixture of 2.02g of (S)-(+)-2-(4-hydroxyphenyl)propionic acid 2-succinimidoethylester ($[\alpha]_D^{20}$ = +57.2° (c=1.00, EtOH)) and 30ml of pyridine. After warming the mixture slowly up to room temperature, it was stirred overnight, and the reaction mixture was concentrated under reduced pressure. Ether was added to the residue by decantation, and then 70ml of water was added. At ice bath temperature, a solution of 2.08g of potassium hydrogencarbonate in 30ml of water was added dropwise to the mixture. After the mixture was stirred for 8 hours at the same temperature, the reaction mixture was let stand overnight in a refrigerator. The deposit crystals were collected by filteration, washed with water, and 2.81g of carbonate was collected. At ice bath temperature, 0.54g of methane sulfonic acid was added to a solution of 2.77g of the carbonate in methanol. Some insoluble substances were filtered, and concentrated under reduced pressure. Diisopropylether was added to the residue, and concentrated under reduced pressure again. Diisopropylether was added to the residue, the deposit crystals were collected by filteration, and washed with diisopropylether. The yield of (S)-(+)-2-[4-(4-guanidino benzoyloxy) phenyl] propionic acid 2-succinimido ethylester • methane sulfonate was 2.87g.

m.p. 100-102°C

$[\alpha]_D^{20}$ = + 25.1° (c=1.00, H2O)

NMR (DMSO-d6) δ

1.41(3H,d), 2.47(3H,s), 2.56(4H,s),
3.4-3.9(3H,m), 4.13(2H,bt), 7.13(2H,d),
7.30(2H,d), 7.35(2H,d), 7.74(4H,bs),
8.10(2H,d), 10.17(1H,s),

enzyme inhibiting activity IC50 of trypsin 3.2 × 10-7

[Exapmle 121]

At ice bath temperature, 5.00g of 4-guanidinobenzoylchloride hydrochloride was added to a mixture of 5.07g of (S)-(+)-2-(4hydroxyphenyl)propionic acid N-methylcarbamoylmethylester ($[\alpha]_D^{20}$ = + 75.3° (c=1.00, EtOH)) and 30ml of pyridine. After warming the mixture slowly up to room temperature, it was stirred overnight, and the reaction mixture was concentrated under reduced pressure. Ether was added to the residue by decantation, and then 150ml of water was added. At ice bath temperature, a solution of 5.83g of potassium hydrogencarbonate in 30ml of water was added dropwise to the mixture. After the mixture was stirred for 3 hours at the same temperature, the reaction mixture was let stand overnight in a refrigerator. The deposit crystals were collected by filteration, washed successively with water and acetone, and 7.08g of carbonate was collected. At ice bath tem perature, 1.55g of methane sulfonic acid was added to a solution of 7.08g of the carbonate in methanol. Some insoluble substances were filtered, and concentrated under reduced pressure. Diisopropylether was added to the residue, and concentrated under reduced pressure again. After diisopropylether was added to the residue, the deposit crystals were collected by filteration, and they were washed with diisopropylether. The yield of (S)-(+)-2-[4-(4-guanidinobenzoyloxy)phenyl]propionic acid N-methylcarbamoyl methyl ester • methane sulfonate was 6.78g.

m.p. 110-113°C

$[\alpha]_D^{20}$ = + 21.3° (c=1.00, H2O) NMR (DMSO-d6) δ

1.47(3H,d), 2.47(3H,s), 2.61(3H,d),
3.7-4.7(1H,bs), 3.96(1H,q), 4.44(2H,bs),
7.17(2H,d), 7.40(2H,d), 7.41(2H,d),
8.11(2H,d), 10.07(1H,s),
enzyme inhibiting activity IC50 of trypsin 2.8 × 10-7

[Exapmle 122]

At ice bath temperature, 1.18g of 4-guanidinobenzoylchloride hydrochloride was added to a mixture of 1.50g of (S)-(+)-2-(4hydroxyphenyl)propionic acid 2,4-dimethylphenoxy carbonylmethyl ester ($[\alpha]_D^{20}$ = + 72.3° (c=1.00, EtOH)) and 20ml of pyridine. After warming the mixture slowly up to room temperature, it was stirred overnight, and the reaction mixture was concentrated under reduced pressure. Ether was added to the residue by decantation, and then 50ml of water was added. At ice bath temperature, a solution of 1.37g of potassium hydrogen carbonate in 20ml of water was added dropwise to the mixture. After the mixture was stirred for 3 hours at the same temperature, the reaction mixture was let stand overnight in a refrigerator. The deposit crystals were collected by filteration, washed with water, and 1.90g of carbonate was collected. At ice bath temperature, 0.36g of methane sulfonic acid was added to a solution of 1.90g of car bonate in acetone. After some insoluble substances were filtered, the filterate was concentrated under reduced pressure, and then hexane was added to the residue. The deposit crystals were collected by filteration, and washed with acetone. The yield of (S)-(+)-2-[4-(4-guanidinobenzoyloxy) phenyl]propionic acid 2,4- dimethylphenoxy carbonylmethyl ester • methane sulfonate was 1.92g.
m.p. 104-107°C
$[\alpha]_D^{20}$ = + 41.0° (c=1.00, H2O)
NMR (DMSO-d6) δ
1.51(3H,d), 2.07(3H,s), 2.27(3H,s),
2.53(3H,s), 4.00(1H,q), 4.97(2H,s),
6.7-7.6(9H,m), 7.81(4H,bs), 8.16(2H,d),
10.23(1H,s)
enzyme inhibiting activity IC50 of trypsin 5.2 × 10-7

[Exapmle 123]

At ice bath temperature, 5.00g of 4-guanidinobenzoylchloride hydrochloride was added to a mixture of 4.32g of (S)-(+)-2-(4hydroxyphenyl)propionic acid methylcarbonylmethyl ester ($[\alpha]_D^{20}$ = + 74.1° (c=1.00, EtOH)) and 30ml of pyridine. After warming the mixture slowly up to room temperature, it was stirred overnight, and the reaction mixture was concentrated under reduced pressure. Ether was added to the residue by decantation, and then 100ml of water was added. At ice bath temperature, a solution of 5.84g of potassium hydrogen carbonate in 30ml of water was added dropwise to the mixture. After the mixture was stirred for 6 hours at the same temperature, the reaction mixture was let stand overnight in a refrigerator. The deposit crystals were collected by filteration, washed successively with water and acetone, and 7.33g of carbonate was collected. At ice bath temperature, 1.54g of methane sulfonic acid was added to a solution of 6.80g of carbonate in acetone. After some insoluble substances were filtered, it was concentrated under reduced pressure, and then ether was added to the residue. The mixture was concentrated under reduced pressure again, and then acetone and ether were added to the residue. The deposit crystals were collected by filteration, and washed with ether. The yield of (S)- (+)-2-[4-(4-guanidinobenzoyloxy) phenyl] propionic acid methylcarbonylmethylester • methane sulfonate was 7.33g.
m.p. 150-152°C
$[\alpha]_D^{20}$ = + 30.9° (c=1.00, H2O)
NMR (DMSO-d6) δ
1.49(3H,d), 2.04(3H,s), 2.50(3H,s),
3.94(1H,q), 4.71(2H,s), 7.16(2H,d),
7.40(2H,d), 7.41(2H,d), 7.77(4H,bs),
8.12(2H,d), 10.22(1H,s)
enzyme inhibitng activity IC50 of trypsin 2.7 × 10-7

[Exapmle 124]

At ice bath temperature, 5.00g of 4-guanidinobenzoylchloride hydrochloride was added to a mixture of 5.52g of (S)-(+)-2-(4-hydroxyphenyl)propionic acid phenylcarbonylmethyl ester ($[\alpha]_D^{20}$ = + 84.6° (c=1.00, EtOH)) and 30ml of pyridine. After warming the mixture slowly up to room temperature, it was stirred overnight, and the reaction mixture was concentrated under reduced pressure. Ether was added to the residue by decantation, and then 150ml of water was added. At ice bath temperature, a solution of 5.83g of potassium hydrogen carbonate in 30ml of water was added dropwise to the mixutre. After the mixture was stirred for 7 hours at the same temperature, the reaction mixture was let stand overnight in a refrigerator. The deposit crystals were collected by filteration, washed successively with water and acetone, and 8.39g of carbonate was collected. At ice bath temperature, 1.65g of methane sulfonic acid was added to a solution of 8.32g of carbonate in acetone. Some insoluble substances were filtered, and the filterate was concentrated wider reduced pressure. Ether was added to the residue, the deposit crystals were collected by filteration, and washed with ether. The yield of (S)-(+)-2-[4-(4-guanidinobenzoyloxy) phenyl] propionic acid phenyl carbonylmethyl ester • methane sulfonate was 8.18g.

m.p. 104-107°C

$[\alpha]_D^{20}$ = + 49.8° (c=1.00, H2O)

NMR (DMSO-d6) δ

1.54(3H,d), 2.53(3H,s), 4.01(1H,q),
5.46(2H,s), 7.0-8.2(15H,m), 8.18(2H,d),
10.27(1H,s)

enzyme inhibiting activity IC50 of trypsin 2.9 × 10-7

The enzyme inhibiting activity(IC50) of compounds obtained from each examples are shown below.

| | trypsin | plasmin |
|---|---|---|
| Example 66 | 3.7 × 10-7 | 6.1 × 10-9 |
| Example 98 | 3.05 × 10-7 | 7 × 10-9 |
| Example 99 | 3.75 × 10-7 | 2.5 × 10-8 |
| Example 69 | 1.4 × 10-7 | 1.9 × 10-8 |
| Example 102 | 2.4 × 10-7 | 2.2 × 10-8 |
| Example 103 | 2.65 × 10-7 | 6.1 × 10-8 |
| Example 73 | 4.0 × 10-7 | 6.2 × 10-9 |
| Example 107 | 2.6 × 10-7 | 9 × 10-9 |
| Example 108 | 2.6 × 10-7 | 3.8 × 10-8 |
| Example 77 | 3.8 × 10-7 | 1.45 × 10-7 |
| Example 112 | 2.8 × 10-7 | 9 × 10-8 |
| Example 113 | 2.8 × 10-7 | 2.8 × 10-7 |
| Example 80 | 3.3 × 10-7 | 2.2 × 10-8 |
| Example 116 | 2.2 × 10-7 | 1.9 × 10-8 |
| Example 117 | 2.3 × 10-7 | 6.6 × 10-8 |

| REFERENCE a known compound(IC50) | trypsin | plasmin | thrombin |
|---|---|---|---|
| FOY | 1.3 × 10-6 | 3.0 × 10-6 | |
| Foipan | 2.8 × 10-7 | 5.2 × 10-8 | |
| FOY-251 | 3.3 × 10-7 | 4.7 × 10-7 | 2.84 × 10-6 |
| Futhan | 2.0 × 10-7 | 2.0 × 10-8 | 3.1 × 10-8 |
| E-3123 | 3.6 × 10-7 | 7.5 × 10-8 | 4 × 10-7 |

[Exapmle 125]

At ice bath temperature, 5.15g of 4-guanidinobenzoylchloride hydrochloride was added to a mixture of 5.73g of 4-(1- amidinoethyl)phenol • methane sulfonate and 20ml of pyridine. The mixture was stirred for 0.5 hours at ice bath temperature, and then stirred overnight at room temperature. The reaction mixture was

concentrated under reduced pressure, and 50ml of water was added to the residue. At ice bath temperature, 9.91g of potassium hydrogen carbonate was added to the mixture. The deposit crystals were collected by filteration, washed successively with water and acetone, and 5.44g of carbonate was collected. At ice bath temperature, 1.35g of methane sulfonic acid was added to a solution of 3.00g of carbonate in 20ml of methanol. Some insoluble substances were filtered, and concentrated under reduced pressure. Acetone was added to the residue, and concentrated under reduced pressure, and then ether was addded to the residue. The deposit crystals were collected by filteration, and washed with ether. The yield of 4-guanidinobenzoic acid 4-(1-amidinoethyl) phenylester • 2 methane sulfonate was 3.36g. This compound had 1/2mol of acetone and 1/8mol of diethylether by NMR and showed very high hygroscopicity.

NMR (DMSO-d6) δ

1.10(3/4H,t), 1.62(3H,d), 2.07(3H,s),
2.52(6H,s), 3.38(1/2H,q), 4.10(1H,q),
7.0-8.1(8H,m), 7.87(4H,bs), 8.88(2H,br),
9.10(2H,br), 10.40(1H,br)

enzyme inhibiting activity IC50 of trypsin 3.2 × 10-7
of thrombin 6.3 × 10-8

The examples for the preparation are shown below.

[Example 126]

This example is a preparation for tablets.

A mixture of 300g of a compound which was prepared from [Example 98], 75g of mild sugar and 29g of potato starch was mixed well. The mixture was placed in the fluidized bed granulator, and granules were collected by spraying a connector. The connector was a 5% aqueous solution containing 20g of hydroxypropyl cellulose. And then 15g of carboxymethyl cellulose calsium as a disintegrator and 11g of magnesium stearate as a lusteror were added to the mixture. After that, the mixture was devided into 150 mg each, and they were pressed and molded to be tablets.

[Example 127]

This example is a preparation for capsules.

| capsules(prescription for one capsule) | |
|---|---|
| a compound prepared from [Example 107]<br>milk suger<br>crystal cellulose<br>talc<br>magnesium stearate | 100.0mg<br>40.0mg<br>12.0mg<br>4.0mg<br>4.0mg |
| total | 160.0mg |
| (These were filled in capsule size 3 of NIKKYOKU CAPSULE) | |

[Example 128]

This is a preparation for injection.

| injection(prescription for one ample) | |
|---|---|
| a compound prepared from [Example 99]<br>NaCl | 10.0mg<br>6.0mg |
| total | 16.0mg |
| (These were adjusted to 2ml with distilled water for injection) | |

[Example 129]

This example is a preparation for dry syrup.

| dry syrup | |
|---|---|
| a compound obtained from [Example 116]<br>D-mannit<br>white singer<br>hydroxy propyl cellulose<br>perfume | 100.0mg<br>380.0mg<br>500.0mg<br>20.0mg<br>a slight amount |
| total | 1000.0mg |

【Table 1】

| Example | A of formula (Ⅲ) | b. p. or m. p. (℃) | NMR (δ) ($CDCl_3$) |
|---|---|---|---|
| 1 | $OCH_3$ | bp151-152(3mmHg) | 1.44(3H, d) 3.62(3H, s) 3.66(1H, q) 6.69(2H, d) 6.92(1H, s) 7.13(2H, d) |
| 2 | $OC_2H_5$ | oil | 1.17(3H, t) 1.44(3H, d) 3.63(1H, q) 4.11(2H, q) 6.3-7.0(1H, bs) 6.71(2H, d) 7.12(2H, d) |
| 3 | $OCH(CH_3)_2$ | mp46-47.5 | 1.11(3H, d) 1.22(3H, d) 1.44(3H, d) 3.60(1H, q) 4.86(1H, qq) 6.72(2H, d) 6.91(1H, bs) 7.10(2H, d) |
| 4 | $OCH_2CF_3$ | oil | 1.47(3H, d) 3.73(1H, q) 4.38(2H, q) 5.91(1H, bs) 6.67(2H, d) 7.08(2H, d) |
| 5 | $OCH_2C_6H_5$ | oil | 1.44(3H, d) 3.69(1H, q) 5.03(2H, s) 6.37(1H, bs) 6.83(2H, d) 7.08(2H, d) 7.19(5H, s) |
| 6 | $OCH_2C_6H_4F(p)$ | mp51-53 | 1.44(3H, d) 3.69(1H, q) 5.01(2H, s) 6.41(1H, s) 6.5-7.4(8H, m) |
| 7 | $OCH_2CH_2$ 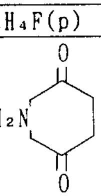 | mp100-102 | 1.41(3H, d) 2.54(4H, s) 3.58(1H, q) 3.73(2H, t) 4.21(2H, t) 6.53(1H, s) 6.71(2H, d) 7.07(2H, d) |
| 8 | $OCH_2\underset{\overset{\|}{O}}{C}N(CH_3)_2$ | mp126-127 | 1.47(3H, d) 2.87(3H, s) 2.90(3H, s) 3.75(1H, q) 4.50(1H, d) 4.77(1H, d) 6.69(2H, d) 7.10(2H, d) 7.53(1H, bs) |
| 9 | $OCH_2\underset{\overset{\|}{O}}{C}NHCH_3$ | oil | 1.49(3H, d) 2.59(3H, d) 3.70(1H, q) 4.33(1H, d) 4.71(1H, d) 5.63(1H, bs) 6.79(2H, d) 7.13(2H, d) 6.5-7.2(1H, br) |
| 10 | $OCH_2\underset{\overset{\|}{O}}{C}NH_2$ | mp78-80 | 1.42(3H, d) 3.77(1H, d) 4.28(1H, d) 4.55(1H, d) 6.74(2H, d) 7.0-7.3(1H, br) 7.15(2H, d) 9.37(2H, s) |
| 11 | $OCH_2\underset{\overset{\|}{O}}{C}N(C_2H_5)_2$ | mp70-72 | 1.14(6H, bt) 1.47(3H, d) 2.9-3.7(4H, m) 3.77(1H, q) 4.53(1H, d) 4.83(1H, d) 6.74(2H, d) 7.14(2H, d) 7.55(1H, s) |
| 12 | $OCH_2\underset{\overset{\|}{O}}{C}NCH_3$ $OCH_2C_6H_5$ | oil | 1.49(3H, d) 2.83(3H, br) 3.77(1H, m) 4.2-4.6(2H, m) 4.55(1H, d) 4.84(1H, |

(note) Example 10 was measured in DMSO-$d_6$.

【Table 2】

| Example | A of formula (Ⅲ) | b.p. or m.p. (℃) | NMR (δ) ($CDCl_3$) |
|---|---|---|---|
| 13 | 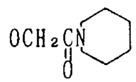 | mp92-94 | 1.47(3H,d) 1.6-2.3(4H,m) 3.0-3.7(4H,m) 3.76(1H,q) 4.48(1H,d) 4.69(1H,d) 6.73(2H,d) 7.11(2H,d) 7.80(1H,s) |
| 14 | 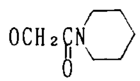 | mp103-105 | 1.46(3H,d) 1.54(6H,bs) 3.22(2H,bs) 3.47(2H,bs) 3.74(1H,q) 4.50(1H,d) 4.93(1H,d) 6.71(2H,d) 7.10(2H,d) 7.66(1H,s) |
| 15 | 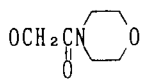 | mp128-130 | 1.42(3H,d) 3.1-4.0(9H,m) 4.71(1H,s) 6.70(2H,d) 7.13(2H,d) 9.14(1H,s) |
| 16 | 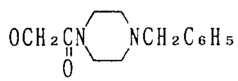 | mp135-137 | 1.50(3H,d) 2.37(4H,m) 3.30(4H,m) 3.50(2H,s) 3.77(1H,q) 4.70(2H,s) 6.70(2H,d) 7.13(2H,d) 7.27(5H,s) |
| 17 | 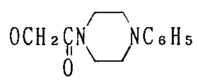 | oil | 1.50(3H,d) 2.9-3.2(4H,m) 3.2-3.9(4H,m) 3.77(1H,q) 4.63(1H,d) 4.77(1H,d) 6.7-7.4(9H,m) |
| 18 | $OCH_2COCH_3$ | oil | 1.34(3H,d) 3.71(3H,s) 3.80(1H,q) 4.62(2H,s) 5.83(1H,bs) 6.76(2H,d) 7.20(2H,d) |
| 19 | $OCH_2COC_2H_5$ | oil | 1.22(3H,t) 1.52(3H,d) 3.77(1H,q) 4.18(2H,q) 4.54(2H,s) 5.44(1H,bs) 6.75(2H,d) 7.20(2H,d) |
| 20 | $OCH_2COC(CH_3)_3$ | mp57-62 | 1.42(9H,s) 1.49(3H,d) 3.71(1H,q) 4.43(2H,s) 5.30(1H,s) 6.69(2H,d) 7.13(2H,d) |
| 21 | $OCH_2COCH_2C_6H_5$ | oil | 1.49(3H,d) 3.78(1H,q) 4.64(2H,s) 5.17(2H,s) 6.05(1H,bs) 6.75(2H,d) 7.18(2H,d) 7.36(5H,s) |

(note) Example 15 was measured in DMSO-$d_6$.

【Table 3】

| Example | A of formula (Ⅲ) | b.p. or m.p. (℃) | NMR (δ) (CDCl$_3$) |
|---|---|---|---|
| 22 | $OCH_2COC_6H_3(CH_3)_2$(o,p)<br>O | mp110-112 | 1.55(3H,d) 2.09(3H,s) 2.27(3H,s) 3.79(1H,q) 4.79(2H,s) 5.09(1H,s) 6.6-7.3(7H,m) |
| 23 | $OCH_2CCH_3$<br>O | oil | 1.50(3H,d) 2.04(3H,s) 3.77(1H,q) 4.92(2H,s) 6.23(1H,bs) 6.73(2H,d) 7.17(2H,d) |
| 24 | $OCH_2CC_6H_5$<br>O | mp117-119 | 1.53(3H,d) 3.81(1H,q) 5.24(2H,s) 5.57(1H,bs) 6.70(2H,d) 7.16(2H,d) 7.1-8.0(5H,m) |
| 25 | $N(C_2H_5)_2$ | mp123-126 | 1.00(3H,t) 1.10(3H,t) 1.40(3H,d) 3.27(2H,q) 3.30(2H,q) 3.78(1H,q) 6.83(2H,d) 7.10(2H,d) 8.53(1H,s) |
| 26 | $NH_2$ | mp144-145 | 1.33(3H,d) 3.48(1H,q) 6.43(1H,bs) 6.63(2H,d) 6.78(1H,bs) 7.06(2H,d) 8.92(1H,s) |
| 27 | $N(CH_3)_2$ | mp180-181 | 1.25(3H,d) 2.83(6H,s) 3.83(1H,q) 6.63(2H,d) 6.99(2H,d) 9.01(1H,s) |
| 28 | 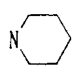 | mp151-153 | 0.7-1.8(6H,m) 1.40(3H,d) 3.33(4H,bt) 3.80(1H,q) 6>80(2H,d) 7.07(2H,d) 8.13(1H,bs) |
| 29 | $NCH_2C_6H_5$<br>$CH_3$ | mp142-144 | 1.44(3H,d) 2.86(3H,br) 3.84(1H,bq) 4.50(1H,d) 4.63(1H,d) 6.77(2H,d) 7.08(2H,d) 7.16(5H,bs) 7.63(1H,bs) |
| 30 | $NC_6H_5$<br>$CH_3$ | mp101-104 | 1.20(3H,d) 3.23(3H,s) 3.57(1H,q) 6.56-7.37(9H,m) 7.63(1H,bs) |

(note) Example 26 and 27 were measured in DMSO-d$_6$.

## Claims

1. A propionic acid derivative shown in the formula (I)

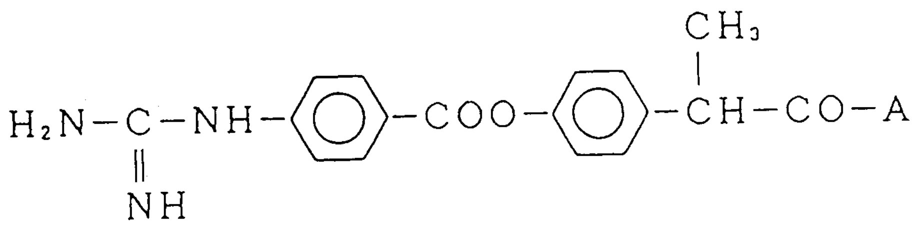

( I )

(In the formula, A is OH, the lower alkoxy of carbon number from one to eight, -N(R1,R2) or the lower alkoxy group of carbon number from one to eight which is substituted with halogen, aryl with or without substituents, group -COB or succinimide group. R1 and R2 is H, the lower alkyl of carbon number from one to eight, arylalkyl with or without subustituents, or a hetero cyclic ring together with the adjacent nitrogen atom. B is OH, the lower alkyl of carbon number from one to eight, aryl with or without substituents, aryloxy with or without substituents, the lower alkoxy of carbon number from one to eight, aralkyloxy with or without substituents or -N(R1,R2). ( here, R1 and R2 are the same as mentioned above. )) or the pharmaceutically acceptable salt thereof.

2. A propionic acid derivative of claim 1 wherein the absolute comfiguration for the position 2 of propionic acid part is R.

3. A propionic acid derivative of claim 1 wherein the absolute comfiguration for the position 2 of propionic acid part is S.

4. A method of the preparation of the propionic acid derivative or the pharmaceutically acceptable salt thereof shown in formula (I) of claim 1, which comprising reacting by esterification between a compound shown in formula (II)

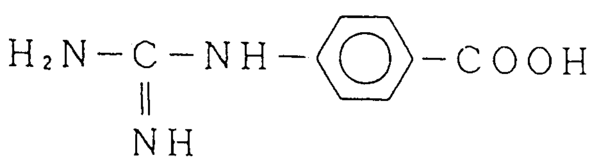

( II )

and propionic ascid or the reactive derivatives thereof shown in formula(III).

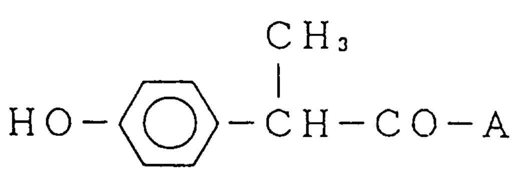

( III )

( A is the same as the definition of formula(I).)

5. A protease inhibitor comprising of said propionic acid derivative or the pharmaceutically acceptable salt thereof as claimed in claim 1.

6. A protease inhibitor as claimed in claim 5 wherein said protease is selected from trypsin, chymotrypsin, plasmin and thrombin.

**7.** A pharmaceutical composition for protease inhibitor which comprises a pharmaceutically acceptable carrier and a propionic acid derivative shown in formula (I) or the pharmaceutically acceptable salt thereof as claimed in claim 1.

**8.** A pharmaceutical composition as claimed in claim 7 wherein said protease is selected from trypsin, chymotrypsin, plasmin and thrombin.

**9.** A method of inhibiting protease in a patient or animal in need of such inhibiting treatment comprising administration to said patient or animal of thrapeutically amount of a compound shown in formula (I) or the pharmaceutically acceptable salt thereof of claim 1.

**10.** A method of inhibiting protease as claimed in claim 9 wherein said protease is selected from trypsin, chymotrypsin, plasmin and thrombin.

**11.** The use of a compound shown in formula (I) or the pharmaceutically acceptable salt thereof as claimed in claim 1 for preparation of a pharmaceutical composition for treatment of inhibiting protease.

**12.** The use of a compound or the pharmaceutically acceptable salt thereof as claimed in claim 11 wherein said protease is selected from trypsin, chymotrypsin, plasmin and thrombin .

**13.** A propionic acid derivative shown in formula (III)

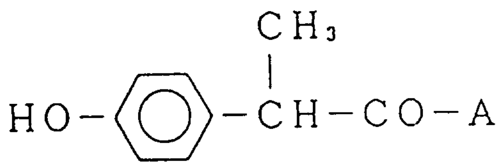

(Ⅲ)

(In the formula, A is OH, the lower alkoxy of carbon number from one to eight, -N(R1,R2) or the lower alkoxy group of carbon number from one to eight which is substituted with halogen, aryl with or without substituents, group -COB or succinimide group. R1 and R2 is H, the lower alkyl of carbon number from one to eight, arylalkyl with or without subustituents, or a hetero cyclic ring together with the adjacent nitrogen atom. B is OH, the lower alkyl of carbon number from one to eight, aryl with or without substituents, aryloxy with or without substituents, the lower alkoxy of carbon number from one to eight, aralkyloxy with or without substituents or -N(R1,R2). ( here, R1 and R2 are the same as mentioned above. )).

**14.** A propionic acid derivative of claim 13 wherein the absolute comfiguration for the position 2 of propionic acid part is R.

**15.** A propionic acid derivative of claim 13 wherein the absolute comfiguration for the position 2 of propionic acid part is S.

## INTERNATIONAL SEARCH REPORT

International application No.
PCT/JP93/01783

**A. CLASSIFICATION OF SUBJECT MATTER**

Int. $Cl^5$ C07C279/18, A61K31/245

According to International Patent Classification (IPC) or to both national classification and IPC

**B. FIELDS SEARCHED**

Minimum documentation searched (classification system followed by classification symbols)

Int. $Cl^5$ C07C279/00, 279/18, A61K31/245, 31/24

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| X | JP, A, 50-4038 (Ono Pharmaceutical Co., Ltd.), January 16, 1975 (16. 01. 75), Pages 1 to 3, (Family: none) | 1-3, 5-15 |
| X | JP, A, 4-21665 (Kazumasa Ikoma), January 24, 1992 (24. 01. 92), Pages 1 to 4, (Family: none) | 1-15 |
| Y | JP, B2, 56-3345 (Ono Pharmaceutical Co., Ltd.), January 24, 1981 (24. 01. 81), Pges 1 to 7, (Family: none) | 1-15 |
| Y | JP, A, 62-111963 (Ono Pharmaceutical Co., Ltd.), May 22, 1987 (22. 05. 87), Pages 1 to 7 & EP, A2, 222,608 | 1-15 |
| A | JP, A, 2-207021 (Ono Pharmaceutical Co., Ltd.), August 16, 1990 (16. 08. 90), Pages 1 to 3, (Family: none) | 1-15 |
| A | JP, A, 2-262516 (Ono Pharmaceutical Co., Ltd.), | 1-15 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| February 17, 1994 (17. 02. 94) | March 8, 1994 (08. 03. 94) |
| Name and mailing address of the ISA/ Japanese Patent Office | Authorized officer |
| Facsimile No. | Telephone No. |